# EUROPEAN PATENT APPLICATION

(11) **EP 1 422 524 A1**
(43) Date of publication of application: **26.05.2004**
(21) Application number: 02765357.5
(22) Date of filing: 27.08.2002
(51) Int. Cl.: G01N 33/50, G01N 33/15, A61K 45/00, A61K 38/16, A61K 39/395, A61P 1/00, A61P 1/16, A61P 3/04, A61P 3/10, A61P 9/00, A61P 25/00, A61P 25/22, A61P 29/00, A61P 35/00, A61P 37/00

(54) **SCREENING METHOD**

(30) Priority: 28.08.2001 JP 2001258479
(71) Applicant: Takeda Chemical Industries, Ltd., Osaka-shi, Osaka 541-0045 (JP)
(72) Inventor: HINUMA, Shuji, Tsukuba-shi, Ibaraki 305-0821 (JP); FUJII, Ryo, Tsukuba-shi, Ibaraki 305-0821 (JP); HABATA, Yugo, Tsukuba-shi, Ibaraki 305-0044 (JP); KAWAMATA, Yuji, Tsukuba-shi, Ibaraki 305-0035 (JP); HOSOYA, Masaki, Tsuchiura-shi, Ibaraki 300-0007 (JP); FUKUSUMI, Shoji, Tsukuba-shi, Ibaraki 305-0044 (JP); KOMATSU, Hidetoshi, Ibaraki 300-4117 (JP)
(74) Representative: Rickard, Timothy Mark Adrian
(86) International application number: PCT/JP2002/008622
(87) International publication number: WO 2003/021262

(57) **Abstract**

Using a G protein-coupled receptor protein (TGR4) or its salt containing the same or substantially the same amino acid sequence as the amino acid sequence represented by SEQ ID NO: 1, and a phospholipid compound, a compound that alters the binding property between the receptor protein or its salt, and the phospholipids compound, or a salt thereof, can be efficiently screened.

## Description

### FIELD OF THE INVENTION

The present invention relates to a screening method using human-derived G protein-coupled receptor protein (TGR4) or its salt, and phospholipids compound as a ligand, a novel rat- and mouse-derived TGR4, a DNA encoding the same and the like.

### BACKGROUND ART

Physiological active substances such as various hormones and neurotransmitters regulate the biological function via specific receptor proteins present on cell membranes. Many of these receptor proteins are coupled with guanine nucleotide-binding protein (hereinafter sometimes referred to as G protein) and mediate the intracellular signal transduction via activation of G protein. These receptor proteins possess the common structure containing seven transmembrane domains and are thus collectively referred to as G protein-coupled receptor proteins or seven-transmembrane receptor proteins (7TMR).

G protein-coupled receptor proteins present on the cell surface of each functional cell and organ in the body, and play important physiological roles as the target of the molecules that regulate the functions of the cells and organs, e.g., hormones, neurotransmitters, physiologically active substances and the like. Receptors transmit signals to cells via binding with physiologically active substances, and the signals induce various reactions such as activation and inhibition of the cells.

To clarify the relationship between substances that regulate complex biological functions in various cells and organs, and their specific receptor proteins, in particular, G protein-coupled receptor proteins, would elucidate the functional mechanisms in various cells and organs in the body to provide a very important means for development of drugs closely associated with the functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or many other physiologically active substances still exist in the body and, as to their receptor proteins, many of these proteins have not yet been reported. In addition, it is still unknown if there are subtypes of known receptor proteins.

It is very important means for development of drugs to clarify the relationship between substances that regulate elaborated functions in vivo and their specific receptor proteins. Furthermore, for efficient screening of agonists and antagonists to receptor proteins and development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to predict their functions from the information.

Although, in WO00/22131 and WO00/23588, the G protein-coupled receptor protein (TGR4) used in the present invention and its DNA have been described, there is no description about a specific ligand to the receptor protein and a function of the receptor protein.

In WO01/77326, there is a description about the G protein-coupled receptor protein (TGR4) used in the present invention and its DNA. Further, lysophosphatidic acid as a ligand, and central nerve diseases (e.g., Alzheimer's disease, dementia, eating disorder, etc.), inflammatory diseases (e.g., allergy, asthma, rheumatism, etc.), circulatory diseases (e.g., hypertension, cardiomegaly, angina, arteriosclerosis, etc.), cancer (e.g., non-small cell lung cancer, ovarian cancer, prostate cancer, stomach cancer, bladder cancer, breast cancer, cervical cancer, colon cancer, rectum cancer, etc.), diabetes, immune system diseases (e.g., AIDS, atopic dermatitis, allergy, asthma, rheumatic arthritis, psoriasis, arteriosclerosis, diabetes, Alzheimer's disease, etc.) and alimentary diseases (e.g., hypersensitivity colitis, ulcerous colitis, Delhi belly, ileus, etc.) as the associated diseases are exemplified.

Substances that inhibit binding between G protein-coupled receptors and physiologically active substances (i.e., ligands), and substances that bind and induce signals similar to those induced by physiologically active substances (i.e., ligands) have been used as pharmaceuticals, as antagonists and agonists specific to the receptors, that regulate the biological functions. Therefore, discovery and gene cloning (e.g., cDNA) of a novel G protein-coupled receptor that can be targeted for pharmaceutical development are very important means in search for a specific ligand, agonist, and antagonist of the novel G protein-coupled receptor.

However, not all G protein-coupled receptors have been discovered. There are unknown G protein-coupled receptors and many of these receptors in which the corresponding ligands are yet unidentified are called orphan receptors. Therefore, search and functional elucidation of a novel G protein-coupled receptor is awaited.

G protein-coupled receptors are useful in searching for a novel physiological active substance (i.e., ligand) using the signal transduction activity as the index and in search for agonists and antagonists of the receptor. Even if no physiological ligand is found, agonists and antagonist of the receptor may be prepared by analyzing the physiological action of the receptor through inactivation experiment of the receptor (knockout animal). Ligands, agonists, antagonists, etc. of the receptor are expected to be used as a medicament for prevention/treatment and diagnosis of diseases associated with dysfunction of the G protein-coupled receptor.

Lowering or accentuation in functions of the G protein-coupled receptor due to genetic aberration of the receptor in vivo causes some disorders in many cases. In this case, the G protein-coupled receptor may be used not only for administration of antagonists or agonists of the receptor, but also for gene therapy by transfer of the receptor gene into the body (or some specific organs) or by introduction of the antisense nucleic acid of the receptor gene into the body (or the specific organ). In the gene therapy, information on the base sequence of the receptor gene is essentially required for investigating deletion or mutation in the gene. The receptor gene is also applicable as a medicament for prevention/treatment and diagnosis of diseases associated with dysfunction of the receptor.

The present invention provides applications of a novel and useful G protein-coupled receptor protein as described above. That is, the present invention provides a novel G protein-coupled receptor protein, its partial peptides and salts thereof, polynucleotides (DNA and RNA, and derivatives thereof) containing the polynucleotides (DNA and RNA, and derivatives thereof) encoding the G protein-coupled receptor protein or its partial peptides, applications of antibodies to the G protein-coupled receptor protein, its partial peptides and salts thereof, as well as methods for screening the compounds that alter the expression level of said G protein-coupled receptor protein, methods for determination of ligands to the G protein-coupled receptor protein, methods for screening the compounds (antagonists and agonists) or salts thereof that alter the binding property of ligands and the G protein-coupled receptor protein, kits for use in the screening methods, compounds (antagonists and agonists) or salts thereof that alter the binding property of ligands obtainable by the screening methods or the screening kits, and medicaments comprising the compounds (antagonists and agonists) that alter the binding property of ligands to the G protein-coupled receptor protein, or compounds or salts thereof that alter the expression level of the G protein-coupled receptor protein.

### DISCLOSURE OF THE INVENTION

As a result of extensive investigations, the present inventors have found that one of the ligands to the G protein-coupled receptor protein (TGR4) derived from human brain, which were described in WO 01/77326 Official Gazette, is a phospholipids compound. Based on these findings, the present inventors have continued further extensive studies and as a result, have come to accomplish the present invention.

Thus, the present invention provides the following features:
[1] A screening method for (1) a G protein-coupled receptor protein containing the same or substantially the same amino acid sequence as the amino acid sequence represented by SEQ ID NO: 1, a partial peptide thereof or salts thereof, and (2) a compound or a salt thereof that alters the binding property between the receptor protein or a salt thereof and phopholipid compound;
[2] The screening method according to [1], wherein the G protein-coupled receptor protein is a protein consisting of the amino acid sequence represented by SEQ ID NO: 1, SEQ ID NO: 8, or SEQ ID NO: 10;
[3] The screening method according to [1], wherein the phospholipid compound is (1) a compound, wherein a pyrophosphate group is bound to the end of repeated structure consisting of at least one isoprene unit, or (2) a compound, wherein a phosphate group is bound to glycerol bone, and thereto a fatty acid or long chain alcohol is bound by ester bond;
[4] The screening method according to [1], wherein the phospholipids compound is geranylgeranyl 2-phosphate (GGPP), farnesyl 2-phosphate (FPP) or lysophophatydinic acid (LPA);
[5] The screening method according to [1], wherein the phospholipids compound is geranylgeranyl 2-phosphate (GGPP) or farnesyl 2-phosphate (FPP);
[6] A screening kit for (1) a G protein-coupled receptor protein containing the same or substantially the same amino acid sequence as the amino acid sequence represented by SEQ ID NO: 1, a partial peptide thereof or salts thereof, and (2) a compound or a salt thereof that alters the binding property between the receptor protein or a salt thereof and phopholipid compound;
[7] A compound or a salt thereof that alters the binding property between phopholipid compound and the receptor protein or a salt thereof containing the same or substantially the same amino acid sequence as the amino acid sequence represented by SEQ ID NO: 1, which is obtainable by using the screening method according to [1] or the screening kit according to [6];
[8] A medicine comprising the compound or a salt thereof that alters the binding property between phopholipid compound and the receptor protein or a salt thereof containing the same or substantially the same amino acid sequence as the amino acid sequence represented by SEQ ID NO: 1;
[9] The medicine according to [8], which is a prophylactic/therapeutic agent for central nerve diseases, inflammatory diseases, circulatory diseases, cancer, diabetes, immune system diseases or alimentary diseases;
[10] The medicine according to [8], which is a prophylactic/therapeutic agent for liver and cholecyst diseases, alimentary diseases, anxiety, pain or obesity;
[11] A determination method of ligand to the G protein-coupled receptor protein or its salt, which comprises measuring intracellular Ca²⁺ concentration increasing activity or intracellular cAMP producing activity when a test compound is brought into contact with a cell containing the G protein-coupled receptor protein containing the same or substantially the same amino acid sequence as the amino acid sequence represented by SEQ ID NO: 1;
[12] A ligand, which is obtained by the method according to [11];
[13] A screening method of agonist or antagonist to the G protein-coupled receptor protein or its salt containing the same or substantially the same amino acid sequence as the amino acid sequence represented by SEQ ID NO: 1, which comprises comparing (i) the case where the G protein-coupled receptor protein containing the same or substantially the same amino acid sequence as the amino acid sequence represented by SEQ ID NO: 1, a partial peptide thereof or salts thereof are brought into contact with (a) phospholipids compound or (b) compound or its salt that alters the binding property between the phospholipids compound and the G protein-coupled receptor protein or its salt, and (ii) the case where the G protein-coupled receptor protein containing the same or substantially the same amino acid sequence as the amino acid sequence represented by SEQ ID NO: 1, a partial peptide thereof or salts thereof are brought into contact with (a) phospholipids compound or (b) compound or its salt that alters the binding property between the phospholipids compound and the G protein-coupled receptor protein or its salt, and a test compound;
[14] A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to the G protein-coupled receptor protein, a partial peptide thereof or salts thereof in the case where (i) (a) labeled phospholipids compound or (b) labeled compound or its salt that alters the binding property between the phospholipids compound and the G protein-coupled receptor protein or its salt is brought into contact with the G protein-coupled receptor protein, a partial peptide thereof or salts thereof, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein, a partial peptide thereof or salts thereof;
[15] A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to a cell containing the G protein-coupled receptor protein, in the case where (i) (a) labeled phospholipids compound or (b) labeled compound or its salt that alters the binding property between the phospholipids compound and the G protein-coupled receptor protein or its salt is brought into contact with the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the cell containing the G protein-coupled receptor protein;
[16] A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to a membrane fraction of the cell containing the G protein-coupled receptor protein, in the case where (i) (a) labeled phospholipids compound or (b) labeled compound or its salt that alters the binding property between the phospholipids compound and the G protein-coupled receptor protein or its salt is brought into contact with the membrane fraction of the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the membrane fraction of the cell containing the G protein-coupled receptor protein;
[17] A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to the G protein-coupled receptor protein, in the case where (i) (a) labeled phospholipids compound or (b) labeled compound or its salt that alters the binding property between the phospholipids compound and the G protein-coupled receptor protein or its salt is brought into contact with the G protein-coupled receptor protein expressing on cell membrane of a transformant, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein expressing on cell membrane of the transformant;
[18] A screening method of agonist or antagonist to the G protein-coupled receptor protein or its salt containing the same or substantially the same amino acid sequence as the amino acid sequence represented by SEQ ID NO: 1, which comprises measuring and comparing intracellular Ca²⁺ concentration increasing activity or intracellular cAMP producing activity, in the case where (i) (a) labeled phospholipids compound or (b) labeled compound or its salt that alters the binding property between the phospholipids compound and the G protein-coupled receptor protein or its salt is brought into contact with the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the cell containing the G protein-coupled receptor protein;
[19] A screening method of agonist or antagonist to the G protein-coupled receptor protein or its salt containing the same or substantially the same amino acid sequence as the amino acid sequence represented by SEQ ID NO: 1, which comprises measuring and comparing intracellular Ca²⁺ concentration increasing activity or intracellular cAMP producing activity, in the case where (i) (a) labeled phospholipids compound or (b) labeled compound or its salt that alters the binding property between the phospholipids compound and the G protein-coupled receptor protein or its salt is brought into contact with the G protein-coupled receptor protein expressing on cell membrane of a transformant, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein expressing on cell membrane of the transformant;
[20] The screening method according to [1], wherein the phospholipid compound is (1) a compound, wherein a pyrophosphate group is bound to the end of repeated structure consisting of at least one isoprene unit, or (2) a compound, wherein a phosphate group is bound to glycerol bone, and thereto a fatty acid or long chain alcohol is bound by ester bond;
[21] The screening method according to [13] through [20], wherein the phospholipids compound is geranylgeranyl 2-phosphate (GGPP), farnesyl 2-phosphate (FPP) or lysophophatidic acid (LPA);
[22] The screening method according to [13] through [20], wherein the phospholipids compound is geranylgeranyl 2-phosphate (GGPP) or farnesyl 2-phosphate (FPP);
[23] The screening method according to [13] through [20], wherein the test compound is a compound designed for binding to the ligand binding pocket based on atom coordinate of the active site of the G protein-coupled receptor protein or its salt containing the same or substantially the same amino acid sequence as the amino acid sequence represented by SEQ ID NO: 1 and the location of ligand binding pocket;
[24] An agonist or an antagonist to the G protein-coupled receptor protein containing the same or substantially the same amino acid sequence as the sequence represented by SEQ ID NO: 1, or a salt thereof, which is obtainable using any screening method according to [13] through [23];
[25] A medicine, which comprises the agonist or the antagonist to the G protein-coupled receptor protein containing the same or substantially the same amino acid sequence as the sequence represented by SEQ ID NO: 1;
[26] A prophylactic/therapeutic agent for central nerve diseases, inflammatory diseases, circulatory diseases, cancer, diabetes, immune system diseases or alimentary diseases, which comprises the agonist or the antagonist to the G protein-coupled receptor protein containing the same or substantially the same amino acid sequence as the sequence represented by SEQ ID NO: 1;
[27] A prophylactic/therapeutic agent for liver and cholecyst diseases, alimentary diseases, anxiety, pain or obesity, which comprises the agonist or the antagonist to the G protein-coupled receptor protein containing the same or substantially the same amino acid sequence as the sequence represented by SEQ ID NO: 1;
[28] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises the G protein-coupled receptor protein containing the same or substantially the same amino acid sequence as the sequence represented by SEQ ID NO: 1, a partial peptide thereof or salts thereof;
[29] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a DNA containing DNA encoding the G protein-coupled receptor protein containing the same or substantially the same amino acid sequence as the sequence represented by SEQ ID NO: 1;
[30] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a polynucleotide containing polynucleotide encoding the G protein-coupled receptor protein containing the same or substantially the same amino acid sequence as the sequence represented by SEQ ID NO: 1;
[31] A diagnostic method for central nerve diseases, inflammatory diseases, circulatory diseases, cancer, diabetes, immune system diseases, liver and cholecyst diseases, alimentary diseases, anxiety, pain or obesity, which comprises using a polynucleotide containing polynucleotide encoding the G protein-coupled receptor protein containing the same or substantially the same amino acid sequence as the sequence represented by SEQ ID NO: 1, or a partial peptide thereof;
[32] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a compound that alters an expression level of the G protein-coupled receptor protein containing the same or substantially the same amino acid sequence as the sequence represented by SEQ ID NO: 1, or a salt thereof;
[33] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises an antibody against the G protein-coupled receptor protein containing the same or substantially the same amino acid sequence as the sequence represented by SEQ ID NO: 1, a partial peptide thereof or salts thereof;
[34] A diagnostic agent for central nerve diseases, inflammatory diseases, circulatory diseases, cancer, diabetes, immune system diseases, liver and cholecyst diseases, alimentary diseases, anxiety, pain or obesity, which comprises an antibody against the G protein-coupled receptor protein containing the same or substantially the same amino acid sequence as the sequence represented by SEQ ID NO: 1, a partial peptide thereof or salts thereof;
[35] A diagnostic method for central nerve diseases, inflammatory diseases, circulatory diseases, cancer, diabetes, immune system diseases, liver and cholecyst diseases, alimentary diseases, anxiety, pain or obesity, which comprises using the quantification method for the G protein-coupled receptor protein using the antibody against the G protein-coupled receptor protein containing the same or substantially the same amino acid sequence as the sequence represented by SEQ ID NO: 1, a partial peptide thereof or salts thereof;
[36] A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof;
[37] A prophylactic/therapeutic method for central nerve diseases, inflammatory diseases, circulatory diseases, cancer, diabetes, immune system diseases, liver and cholecyst diseases, alimentary diseases, anxiety, pain or obesity, which comprises administering to mammals an effective amount of (a) the agonist or the antagonist to the G protein-coupled receptor protein containing the same of substantially the same amino acid sequence as the sequence represented by SEQ ID NO: 1, or a salt thereof, (b) the G protein-coupled receptor protein containing the same or substantially the same amino acid sequence as the sequence represented by SEQ ID NO: 1, a partial peptide thereof or salts thereof, (c) a polynucleotide containing a polynucleotide encoding the G protein-coupled receptor protein containing the same or substantially the same amino acid sequence as the sequence represented by SEQ ID NO: 1, or a partial peptide thereof, (d) an antibody against the G protein-coupled receptor protein containing the same or substantially the same amino acid sequence as the sequence represented by SEQ ID NO: 1, a partial peptide thereof or salts thereof, or (e) a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof;
[38] Use of (a) the agonist or the antagonist to the G protein-coupled receptor protein containing the same or substantially the same amino acid sequence as the sequence represented by SEQ ID NO: 1, or a salt thereof, (b) the G protein-coupled receptor protein containing the same or substantially the same amino acid sequence as the sequence represented by SEQ ID NO: 1, a partial peptide thereof or salts thereof, (c) a polynucleotide containing a polynucleotide encoding the G protein-coupled receptor protein containing the same or substantially the same amino acid sequence as the sequence represented by SEQ ID NO: 1, or a partial peptide thereof, (d) an antibody against the G protein-coupled receptor protein containing the same or substantially the same amino acid sequence as the sequence represented by SEQ ID NO: 1, a partial peptide thereof or salts thereof, or (e) a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof for manufacturing a prophylactic/therapeutic agent for central nerve diseases, inflammatory diseases, circulatory diseases, cancer, diabetes, immune system diseases, liver and cholecyst diseases, alimentary diseases, anxiety, pain or obesity;
[39] A G protein-coupled receptor protein consisting of the amino acid sequence represented by SEQ ID NO: 8 or SEQ ID NO: 10, a partial peptide thereof or salts thereof;
[40] A polynucleotide containing the polypeptide encoding the G protein-coupled receptor protein according to [39];
[41] A DNA consisting of the base sequence represented by SEQ 8 or SEQ ID NO: 10;
[42] A recombinant vector containing the polynucleotide according to [40];
[43] A transformant, which is transformed with the recombinant vector according to [42];
[44] A manufacturing method of the G protein-coupled receptor protein according to [39], or a salt thereof, which comprises culturing the transformant according to [43] and producing the G protein-coupled receptor protein according to [39], or a salt thereof;
[45] A medicine, which comprises the G protein-coupled receptor protein according to [39], a partial peptide thereof or salts thereof;
[46] A medicine, which comprises the polynucleotide according to [40];
[47] A diagnostic agent, which comprises the polynucleotide according to [40];
[48] An antibody against the G protein-coupled receptor protein according to [39], a partial peptide thereof or salts thereof;
[49] The antibody according to [48], which is a neutralizing antibody that inactivates signal transduction of the G protein-coupled receptor protein according to [39];
[50] A diagnostic agent, which comprises the antibody according to [48];
[51] A medicine, which comprises the antibody according to [48];
[52] An antisense polynucleotide, which comprises a complementary base sequence to the polynucleotide according to [40] or a portion thereof;
[53] A diagnostic agent, which comprises the antisense polynucleotide according to [52];
[54] A medicine, which comprises the antisense polynucleotide according to [52];
[55] A screening method for the agonist or the antagonist to the G protein-coupled receptor protein according to [39], which comprises using the G protein-coupled receptor protein according to [39], a partial peptide thereof or salts thereof;
[56] A screening kit for the agonist or the antagonist to the G protein-coupled receptor protein according to [39], which comprises the G protein-coupled receptor protein according to [39], a partial peptide thereof or salts thereof;
[57] An agonist or an antagonist to the G protein-coupled receptor protein according to [39], which is obtained using the screening method according to [55] or the screening kit according to [56];
[58] A medicine, which comprises an agonist or an antagonist to the G protein-coupled receptor protein according to [39];
[59] A screening method for a compound that alters an expression level of the G protein-coupled receptor protein according to [39], or a salt thereof, which comprises using the polynucleotide according to [40];
[60] A screening kit for a compound that alters an expression level of the G protein-coupled receptor protein according to [39], or a salt thereof, which comprises the polynucleotide according to [40];
[61] A compound that alters an expression level of the G protein-coupled receptor protein according to [39], or a salt thereof, which is obtained using the screening method according to [59] or the screening kit according to [60];
[62] A medicine, which comprises a compound that alters an expression level of the G protein-coupled receptor protein according to [39], or a salt thereof.
   The present invention further provides the following features:
[63] The screening method according to [1], wherein the receptor protein is a protein containing a) the amino acid sequence shown by SEQ ID NO: 1, of which at least 1 or 2 (preferably approximately 1 to 30, more preferably approximately 1 to 10, furthermore preferably several (1 to 5)) amino acids are deleted, b) the amino acid sequence shown by SEQ ID NO: 1, to which at least 1 or 2 (preferably approximately 1 to 30, more preferably approximately 1 to 10, furthermore preferably several (1 to 5)) amino acids are added; c) the amino acid sequence shown by SEQ ID NO: 1, in which at least 1 or 2 (preferably approximately 1 to 30, more preferably approximately 1 to 10, furthermore preferably several (1 to 5)) amino acids are substituted to other amino acids; or d) the amino acid sequence containing a combination of these amino acid sequences;
[64] The screening kit according to [6], which is characterized by comprising cells containing the G protein-coupled receptor protein according to [1];
[65] The screening kit according to [6], which is characterized by comprising membrane fractions of cells containing the G protein-coupled receptor protein according to [1]; and
[66] The screening kit according to [6], which is characterized by comprising the G protein-coupled receptor protein, which is expressed on cell membrane of transformant transformed with a recombinant vector harboring a DNA containing DNA encoding the G protein-coupled receptor protein according to [1] by culturing the transformant.

### BRIEF DESCRIPTION OF THE DRAWINGS

FIG. 1 shows a hydrophobicity plot of TGR4.
FIG. 2 shows an amino acid sequence of SEQ ID NO: 1 represented by single letter symbols.
FIG. 3 shows an expression distribution of TGR4 in respective tissues.
FIG. 4 shows an increase of luciferase activity in CHO-mock cells, in which the TGR4 expression vector was introduced, by phospholipids compound. Lateral axis indicates concentration of phospholipids compound. GGPP, FPP and LPA represent geranylgeranyl 2-phosphate, farnesyl 2-phosphate and lysophosphatidylic acid, respectively. The "base" shows a result for no addition of phospholipids compound. Vertical axis indicates luciferase activity.
FIG. 5 shows an increase of intracellular calcium ion mobilization activity in CHO-mock cells by addition of FPP (farnesyl 2-phosphate) or LPA (lysophosphatidylic acid). Lateral axis indicates time (seconds). Vertical axis indicates changes of calcium ion concentration. Diamond, square and triangle show no addition, FPP addition and LPA addition, respectively.
FIG. 6 shows an increase of intracellular calcium ion mobilization activity in TGR4 expressing CHO-mock cells by addition of FPP (farnesyl 2-phosphate) or LPA (lysophosphatidylic acid). Lateral axis indicates time (seconds). Vertical axis indicates changes of calcium ion concentration. Diamond, square and triangle show no addition, FPP addition and LPA addition, respectively.
FIG. 7 shows an increase of intracellular cAMP production in CHO-mock cells by addition of FPP (farnesyl 2-phosphate) or LPA (lysophosphatidylic acid). N=3. Base, FPP and LPA show no addition, FPP addition and LPA addition, respectively. Vertical axis indicates level of cAMP production.
FIG. 8 shows an increase of intracellular cAMP production in TGR4 expressing CHO-mock cells by addition of FPP (farnesyl 2-phosphate) or LPA (lysophosphatidylic acid). N=3. Base, FPP and LPA show no addition, FPP addition and LPA addition, respectively. Vertical axis indicates level of cAMP production.
FIG. 9 shows the base sequence of cDNA encoding rat TGR4.
FIG. 10 shows the amino acid sequence of rat TGR4.
FIG. 11 shows the base sequence of cDNA encoding mouse TGR4.
FIG. 12 shows the amino acid sequence of mouse TGR4.
FIG. 13 shows an expression distribution of human TGR4 mRNA. Vertical axis represents copy numbers of human TGR4 mRNA per 1 ng of poly(A)+ RNA. Lateral axis represents tissues analyzed.
FIG. 14 shows an expression distribution of human TGR4 mRNA. Vertical axis represents copy numbers of human TGR4 mRNA per 1 µl of poly(A)+ RNA. Lateral axis represents cells and tissues analyzed.
FIG. 15 shows an expression distribution of rat TGR4 mRNA. Vertical axis represents copy numbers of rat TGR4 mRNA per 1 ng of poly(A)+ RNA. Lateral axis represents tissues analyzed.

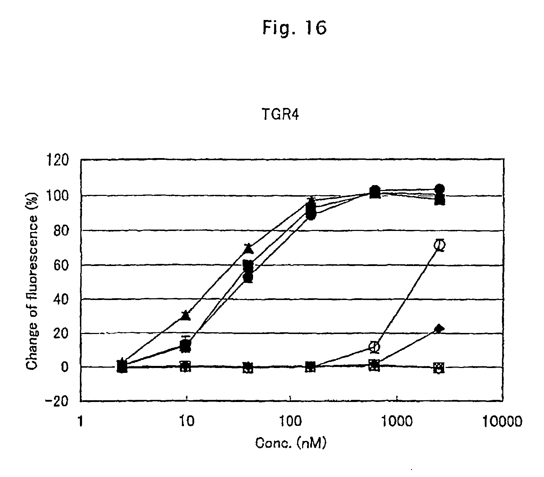

FIG. 16 shows a result for effects of various phospholipids compounds on intracellular calcium ion mobilization activity in TGR4 expressing CHO cells. The term "Conc. (nM)" in lateral axis means concentration of various phospholipids compounds. Closed triangle, closed square, closed diamond and closed circle represent geranylgeranyl 2-phosphate, farnesyl 2-phosphate, geranyl 2-phosphate and lysophosphatidylic acid, respectively. Circle, triangle, square, diamond and x represent sphingosine 1-phosphate, nordeoxycholic acid, deoxycholic acid, geranylgeraniol and farnesol, respectively. The term "change of fluorescence (%)" means the calcium ion mobilization activity. For calculation, results were represented by percents to the reaction where 2 x 10⁻⁶ M farnesyl 2-phosphate was added to the CHO-TGR4. This value is means of n=3.

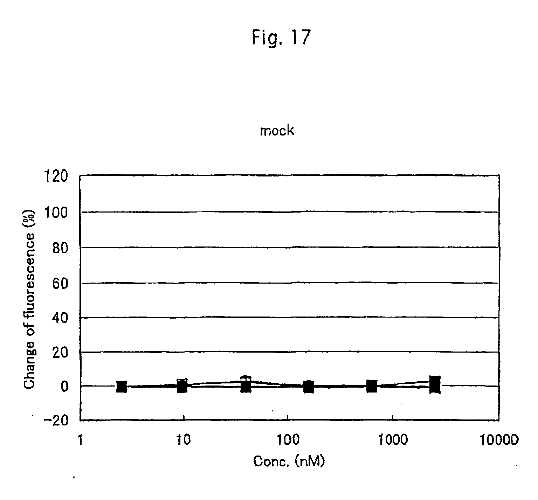

FIG. 17 shows a result for effects of various phospholipids compounds on intracellular calcium ion mobilization activity in mock CHO cells. The term "Conc. (nM)" in lateral axis means concentration of various phospholipids compounds. Closed triangle, closed square, closed diamond and closed circle represent geranylgeranyl 2-phosphate, farnesyl 2-phosphate, geranyl 2-phosphate and lysophosphatidylic acid, respectively. Circle, triangle, square, diamond and x represent sphingosine 1-phosphate, nordeoxycholic acid, deoxycholic acid, geranylgeraniol and farnesol, respectively. The term "change of fluorescence (%)" means the calcium ion mobilization activity. For calculation, results were represented by percents to the reaction where 2 x 10⁻⁶ M farnesyl 2-phosphate was added to the CHO-TGR4. This value is means of n=3.

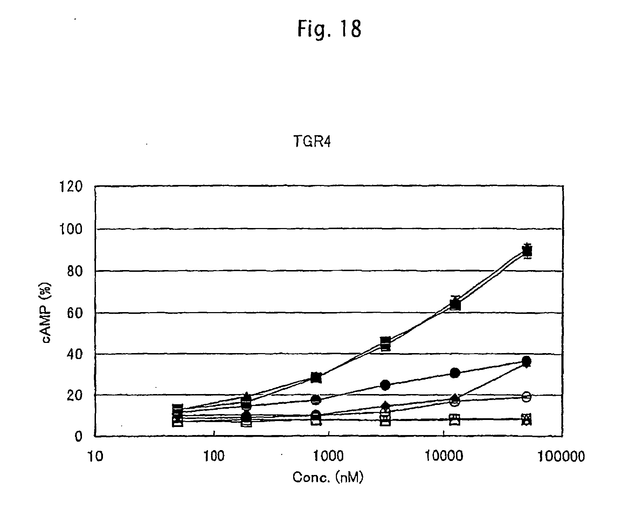

FIG. 18 shows a result for effects of various phospholipids compounds on cAMP production increasing activity in TGR4 expressing CHO cells. The term "Conc. (nM)" in lateral axis means concentration of various phospholipids compounds. Closed triangle, closed square, closed diamond and closed circle represent geranylgeranyl 2-phosphate, farnesyl 2-phosphate, geranyl 2-phosphate and lysophosphatidylic acid, respectively. Circle, triangle, square, diamond and x represent sphingosine 1-phosphate, nordeoxycholic acid, deoxycholic acid, geranylgeraniol and farnesol, respectively. Vertical axis indicates the cAMP production increasing activity. The cAMP production increasing activity was calculated as percents to the reaction where 115 x 10⁻⁶ M farnesyl 2-phosphate was added to the CHO-TGR4. This value is means of n=3.

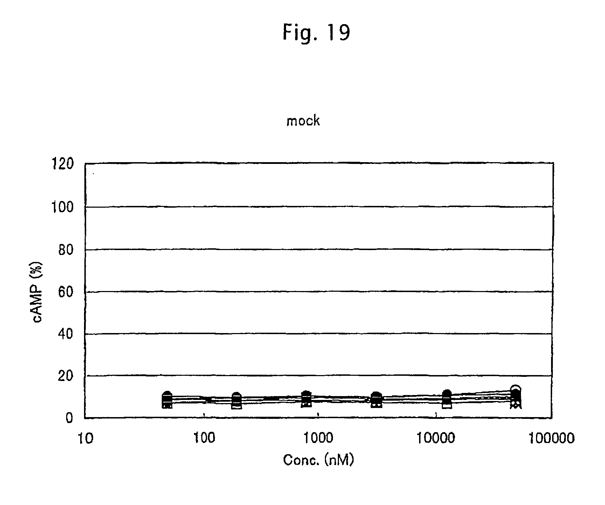

FIG. 19 shows a result for effects of various phospholipids compounds on cAMP production increasing activity in mock CHO cells. The term "Conc. (nM)" in lateral axis means concentration of various phospholipids compounds. Closed triangle, closed square, closed diamond and closed circle represent geranylgeranyl 2-phosphate, farnesyl 2-phosphate, geranyl 2-phosphate and lysophosphatidylic acid, respectively. Circle, triangle, square, diamond and x represent sphingosine 1-phosphate, nordeoxycholic acid, deoxycholic acid, geranylgeraniol and farnesol, respectively. Vertical axis indicates the cAMP production increasing activity. The cAMP production increasing activity was calculated as percents to the reaction where 115 x 10⁻⁶ M farnesyl 2-phosphate was added to the CHO-TGR4. This value is means of n=3.

### BEST MODE FOR CARRYING OUT THE INVENTION

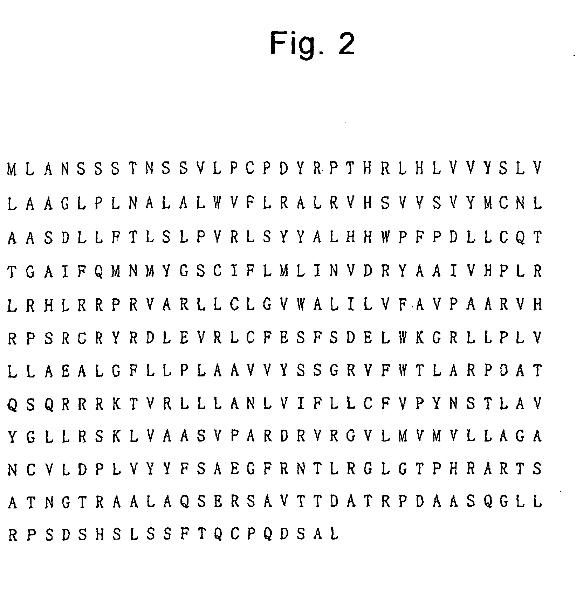

The G protein-coupled receptor protein of the present invention (hereinafter sometimes merely referred to as the receptor protein) is a receptor protein, which contains the same or substantially the same amino acid sequence as the amino acid sequence shown by SEQ ID NO: 1 (FIG. 2).

The receptor protein of the present invention may be any protein derived from any cells (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, pituitary, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testis, ovary, placenta, uterus, bone, joint, skeletal muscle, etc. from human or mammals (e.g., guinea pigs, rats, mice, rabbits, swine, sheep, bovine, monkeys, etc.). The receptor protein may also be a synthetic protein.

The amino acid sequence, which is substantially the same amino acid sequence as that represented by SEQ ID NO: 1, includes an amino acid sequence having at least about 50% homology, preferably at least about 60% homology, more preferably at least about 70% homology, much more preferably at least about 80% homology, among others preferably at least about 90% homology and most preferably at least about 95% homology, to the amino acid sequence represented by SEQ ID NO: 1.

Examples of the protein which contains substantially the same amino acid sequence as that shown by SEQ ID NO: 1 include a protein having substantially the same amino acid sequence as that shown by SEQ ID NO: 1 and having the activity substantially equivalent to that of the amino acid sequence represented by SEQ ID NO: 1, etc.

Examples of the substantially equivalent activity include a ligand binding activity, a signal transduction activity, etc. The term "substantially equivalent" is used to mean that the nature of the activity is the same. Therefore, although it is preferred that activities such as the ligand binding and signal transduction activities, etc. be equivalent (e.g., about 0.01- to about 100-fold, preferably about 0.5- to about 20-fold, more preferably about 0.5- to about 2-fold), quantitative factors such as a level of the activity, a molecular weight of the protein, etc. may differ.

The activities such as ligand binding and signal transduction activities or the like can be determined according to a publicly known method, and also for example, by the ligand determination methods or the screening methods that will be later described.

Proteins containing the following amino acid sequences are used as the receptor protein of the present invention: a) amino acid sequences represented by SEQ ID NO: 1, wherein at least 1 or 2 amino acids (preferably approximately 1 to 30 amino acids, more preferably approximately 1 to 10 amino acids, most preferably several (1 to 5) amino acids) are deleted; b) amino acid sequences represented by SEQ ID NO: 1, to which at least 1 or 2 amino acids (preferably approximately 1 to 30 amino acids, more preferably approximately 1 to 10 amino acids, and most preferably several (1 to 5) amino acids) are added; c) amino acid sequences represented by SEQ ID NO: 1, in which at least 1 or 2 amino acids (preferably approximately 1 to 30 amino acids, more preferably approximately 1 to 10 amino acids, and most preferably several (1 to 5) amino acids) are substituted by other amino acids; or d) combination of the amino acid sequences described in the above.

Throughout the present specification, the receptor proteins are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO: 1, the C-terminus is usually in the form of a carboxyl group (-COOH) or a carboxylate (-COO⁻) but may be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; and the like. In addition, pivaloyloxymethyl or the like, which is used widely as an ester for oral administration, may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the C-terminus described above.

Furthermore, examples of the receptor protein of the present invention include variants of the above receptor proteins, wherein the amino group at the N-terminal methionine residue of the protein supra is protected with a protecting group (for example, a C₁₋₆ acyl group such as a C₂₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₂₋₆ alkanoyl group, e.g., formyl group, acetyl group, etc.), or conjugated proteins such as glycoproteins bound to sugar chains.

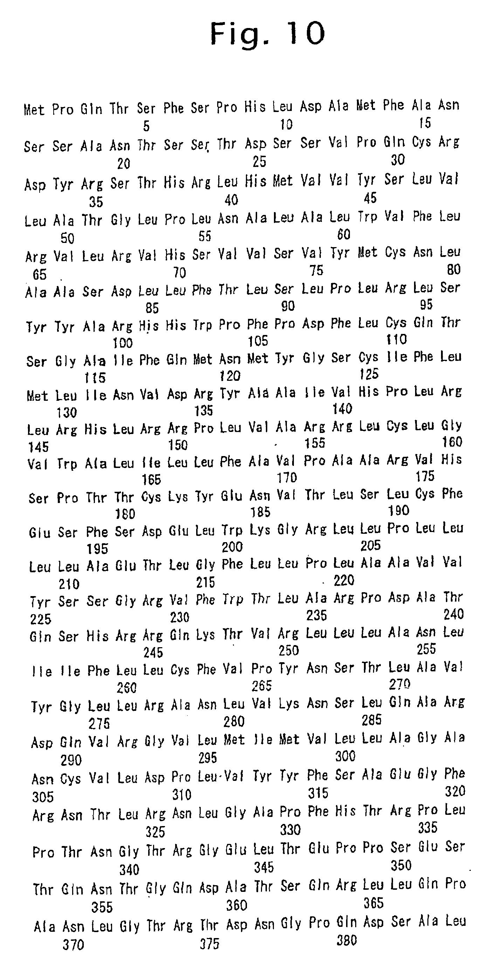

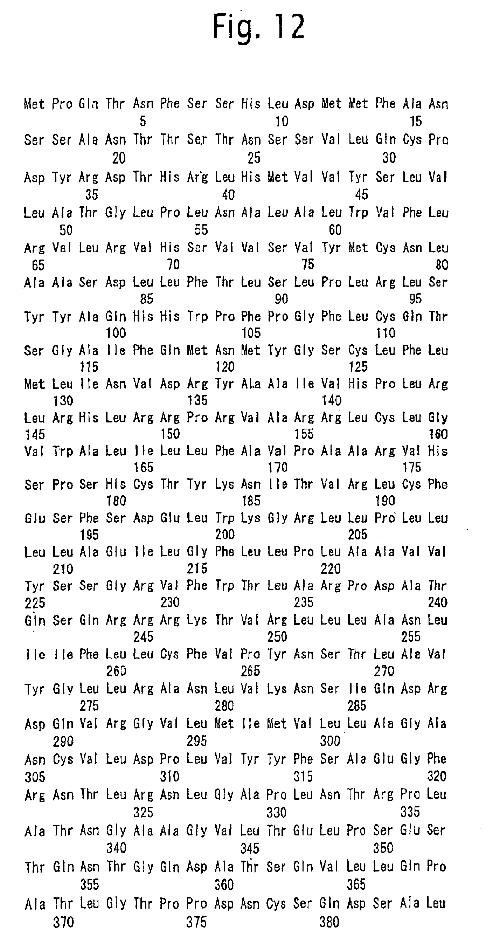

Specific examples of the receptor protein of the present invention which can be used include a G protein-receptor protein (human TGR4) consisting of an amino acid sequence represented by SEQ ID NO: 1, a G protein-receptor protein (rat TGR4) consisting of an amino acid sequence represented by SEQ ID NO: 8 (FIG 10), a G protein-receptor protein (mouse TGR4) consisting of an amino acid sequence represented by SEQ ID NO: 10 (FIG 12), and the like. Among them, rat TGR4 and mouse TGR4 are a novel protein.

As partial peptides of the receptor protein of the present invention (hereinafter sometimes referred to as the partial peptides), any partial peptide can be used so long as it can be a partial peptide of the receptor protein described above. Among the receptor protein molecules of the present invention, for example, those having a site exposed to the outside of a cell membrane and having a substantially equivalent receptor binding activity can be used.

Specifically, the partial peptide of the receptor protein having the amino acid sequence represented by SEQ ID NO: 1, SEQ ID NO: 8 or SEQ ID NO: 10 is a peptide containing the portion analyzed to be extracellular domains (hydrophilic domains) in the hydrophobic plotting analysis. A peptide containing a hydrophobic domain in part can be used as well. In addition, the peptide may contain each domain separately or plural domains together.

In the receptor protein of the present invention, preferred partial peptides are those having at least 20, preferably at least 50, and more preferably at least 100 amino acids, in the amino acid sequence which constitutes the receptor protein of the present invention.

Substantially the same amino acid sequence includes an amino acid sequence having at least about 50% homology, preferably at least about 60% homology, more preferably at least about 70% homology, much more preferably at least about 80% homology, among others preferably at least about 90% homology and most preferably at least about 95% homology, to these amino acid sequences.

Herein, the term "receptor activity substantially equivalent" refers to the same significance as defined above. The "receptor activity substantially equivalent" can be assayed in the same manner as given above.

The partial peptide of the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; or, in which at least 1 or 2 amino acids (preferably approximately 1 to 10 amino acids, more preferably several and most preferably approximately 1 to 5 amino acids) are substituted by other amino acids.

In the partial peptide of the present invention, the C-terminus is normally a carboxyl group (-COOH) or carboxylate (-COO⁻) but the C-terminus may be in the form of an amide (-CONH₂) or an ester (-COOR), as has been described with the protein of the present invention. Where the partial peptide of the present invention contains a carboxyl group (or a carboxylate) at a position other than the C-terminus, it may be amidated or esterified and such an amide or ester is also included within the partial peptide of the present invention. The ester group may be the same group as that described with respect to the C-terminus described above.

As in the receptor protein of the present invention described above, the partial peptide of the present invention further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycopeptides, to which sugar chains are bound, and the like.

For salts of the receptor protein or the partial peptide of the present invention, preferred are salts with physiologically acceptable acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The receptor protein of the present invention or salts thereof may be manufactured by a publicly known method used to purify a receptor protein from human or mammalian cells or tissues described above, or by culturing a transformant that contains the DNA encoding the receptor protein of the present invention, as will be later described. Furthermore, the receptor protein or its salts may also be manufactured by the methods for synthesizing proteins or by modifications thereof, which will also be described hereinafter.

Where the receptor protein or its salts are manufactured from human or mammalian tissues or cells, human or mammalian tissues or cells are homogenized, then extracted with an acid or the like, and the extract can be isolated and purified by a combination of chromatography techniques such as reverse phase chromatography, ion exchange chromatography, and the like.

To synthesize the receptor protein of the present invention, its partial peptide, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, tertiary pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, tertiary butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. Both proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts in the protein of the present invention can be manufactured by publicly known methods for peptide synthesis, or by cleaving the protein of the present invention with an appropriate peptidase. For the methods for peptide synthesis, for example; either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in a) - e) below:
a) M. Bodanszky & M.A. Ondetti: Peptide Synthesis, Interscience Publishers, New York (1966);
b) Schroeder & Luebke: The Peptide, Academic Press, New York (1965);
c) Nobuo Izumiya, et al.: *Peptide Gosei-no-Kiso to Jikken* (Basics and experiments of peptide synthesis), published by Maruzen Co. (1975);
d) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977);
e) Haruaki Yajima, ed.: *Zoku Iyakuhin no Kaihatsu* (A sequel to Development of Pharmaceuticals), Vol. 14, Peptide Synthesis, published by Hirokawa Shoten.

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the receptor protein of the present invention may be any polynucleotide so long as it contains the base sequence (DNA or RNA, preferably DNA) encoding the receptor protein of the present invention described above. Such a polynucleotide may also be any one of DNA encoding the receptor protein of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the receptor protein of the present invention can be quantified by, for example, the publicly known method published in separate volume of *Jikken Igaku* 15 (7) "New PCR and its application" (1997), or by its modifications.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be any DNA having the base sequence shown by SEQ ID NO: 2, or the base sequence hybridizable to the base sequence represented by SEQ ID NO: 2 under highly stringent conditions and encoding a receptor protein having the activities substantially equivalent to those of the receptor protein of the present invention (e.g., a ligand binding activity, a signal transduction activity, etc.).

Specific examples of the DNA hybridizable to the base sequence represented by SEQ ID NO: 2 include DNA containing a base sequence having at least about 70% homology, preferably at least about 80% homology, more preferably at least about 90% homology and most preferably at least about 95% homology, to the base sequence represented by SEQ ID NO: 2.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, for the DNA encoding the receptor protein consisting of the amino acid sequence represented by SEQ ID NO: 1, there may be employed DNA consisting of the base sequence represented by SEQ ID NO: 2; for the DNA encoding the receptor protein consisting of the amino acid sequence represented by SEQ ID NO: 8, there may be employed DNA consisting of the base sequence represented by SEQ ID NO: 9; and for the DNA encoding the receptor protein consisting of the amino acid sequence represented by SEQ ID NO: 10, there may be employed DNA consisting of the base sequence represented by SEQ ID NO: 11.

The polynucleotide comprising a part of the base sequence of the DNA encoding the receptor protein of the present invention or a part of the base sequence complementary to the DNA is used to mean to embrace not only the DNA encoding the partial peptide of the present invention described below but also RNA.

According to the present invention, antisense polynucleotides (nucleic acids) that can inhibit the replication or expression of G protein-coupled receptor protein genes can be designed and synthesized based on the base sequence information of the cloned or determined DNA encoding the G protein-coupled receptor protein. Such a polynucleotide (nucleic acid) is capable of hybridizing to RNA of G protein-coupled receptor protein gene to inhibit the synthesis or function of said RNA or capable of modulating or controlling the expression of a G protein-coupled receptor protein gene via interaction with G protein-coupled receptor protein-associated RNA. Polynucleotides complementary to the selected sequences of RNA associated with G protein-coupled receptor protein and polynucleotides specifically hybridizable to the G protein-coupled receptor protein-associated RNA are useful in modulating or controlling the expression of a G protein-coupled receptor protein gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the G protein-coupled receptor protein genes, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the G protein-coupled receptor protein genes.

The relationship between the targeted nucleic acids and the polynucleotides complementary to at least a part of the target, specifically the relationship between the target and the polynucleotides hybridizable to the target, can be denoted to be "antisense". Examples of the antisense polynucleotides include polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory activity of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) DNA having a partial base sequence of the DNA having the base sequence represented by SEQ ID NO: 2, or (2) any DNA containing a partial base sequence of the DNA having a base sequence hybridizable to the base sequence represented by SEQ ID NO: 2 under highly stringent conditions and encoding a receptor protein which has the activities (e.g., a ligand-biding activity, a signal transduction activity, etc.) substantially equivalent to those of the receptor protein peptide of the present invention.

Specific examples of the DNA that is hybridizable to the base sequence represented by SEQ ID NO: 2 include DNA containing a base sequence having at least about 70% homology, preferably at least about 80% homology, more preferably at least about 90% homology and most preferably at least about 95% homology, to the base sequence represented by SEQ ID NO: 2.

For cloning of the DNA that completely encodes the receptor protein of the present invention or its partial peptide (hereinafter sometimes collectively referred to as the receptor protein of the present invention), the DNA may be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the receptor protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the Gapped duplex method or the Kunkel method or its modification by using a publicly known kit available as Mutan™-G or Mutan™-K (both manufactured by Takara Shuzo Co., Ltd.).

The cloned DNA encoding the receptor protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the receptor protein of the present invention can be manufactured, for example, by (a) excising the desired DNA fragment from the DNA encoding the receptor protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB 110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λP_{L} promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P 10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary, a signal sequence that matches with a host is added to the N-terminus of the receptor protein of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the receptor protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of silkworm can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the G protein-coupled receptor protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogen phosphate, magnesium chloride, etc. In addition, yeast extract, vitamins, growth promoting factors etc. may also be added to the medium. Preferably, pH of the medium is adjusted to about 5 to about 8.

A preferred example of the medium for cultivation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultivated generally at about 30°C to about 40°C for about 6 hours to about 24 hours. If necessary, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the culture is performed at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The culture is usually done at about 30°C to about 40°C for about 15 hours to about 60 hours and, if necessary, the culture can be aerated or agitated.

As described above, the G protein-coupled receptor protein of the present invention can be produced into the cell, in the cell membrane or out of the cell of the transformant.

The receptor protein of the present invention can be separated and purified from the culture described above by the following procedures.

When the protein of the present invention is extracted from the cultured bacteria or cells, after cultivation these cells are collected by a publicly known method and suspended in a appropriate buffer. The bacteria or cells are then disrupted by publicly known methods such as ultrasonication, a treatment with lysozyme and/or freeze-thaw cycling, followed by centrifugation, filtration, etc. Thus, the crude extract of the receptor protein of the present invention can be obtained. The buffer used for the procedures may contain a protein modifier such as urea or guanidine hydrochloride, or a surfactant such as Triton X-100™, etc. When the receptor protein is secreted in the culture, after completion of the cultivation the supernatant can be separated from the bacteria or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the receptor protein thus obtained is in a free form, it can be converted into the salt form by publicly known methods or modifications thereof. On the other hand, when the receptor protein is obtained in the salt form, it can be converted into the free form or in the form of a different salt by publicly known methods or modifications thereof.

The protein of the present invention produced by the recombinant can be treated, prior to or after the purification, with an appropriate protein modifying enzyme so that the receptor protein can be appropriately modified and partially removed a polypeptide. Examples of the protein-modifying enzyme include trypsin, chymotrypsin, arginyl endopeptidase, protein kinase, glycosidase or the like.

The activity of the thus produced receptor protein of the present invention or salts thereof can be determined by a binding experiment to a labeled ligand, by an enzyme immunoassay using a specific antibody.

Antibodies to the receptor protein of the present invention, its partial peptides, or salts thereof may be any of polyclonal antibodies and monoclonal antibodies, as long as they are capable of recognizing the receptor protein of the present invention, its partial peptides, or salts thereof.

The antibodies to the receptor protein of the present invention, its partial peptides, or salts thereof (hereinafter sometimes merely referred to as the receptor protein of the present invention) may be manufactured by publicly known methods for manufacturing antibodies or antisera, using as antigens the receptor protein of the present invention.

### [Preparation of monoclonal antibody]

### (a) Preparation of monoclonal antibody-producing cells

The receptor protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas.
Measurement of the antibody titer in antisera may be made, for example, by reacting a labeled form of the receptor protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the receptor protein etc. as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, a complex of immunogen (receptor protein as an antigen) and a carrier protein is prepared, and a mammal is immunized with the complex in a manner similar to the method described above for the manufacture of monoclonal antibodies. The product containing the antibody to the receptor protein of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The ligand to the receptor protein or its salt of the present invention is a phospholipids compound. For example, (1) a compound wherein a pyrophosphate group is bound to the terminus of repeated structure consisting of one or more isoprene units, or (2) a compound wherein one phosphate group is bound to glycerol backbone and thereto one fatty acid or long chain alcohol is bound via ester bond, is used.

The repeat of isoprene unit is at least one, preferably one to six, more preferably two to four.

As fatty acid, for example, fatty acid containing 6 to 30 carbons is preferred. Specifically, lauric acid, myristic acid, palmitic acid, stearic acid, arachidonic acid, oleic acid, linoleic acid, linolenic acid and the like, are utilized.

As long chain alcohol, for example, alcohol containing 6 to 30 carbons is preferred. Specifically, tetradecanol, pentadecanol, hexadecanol, octadecanol and the like, are utilized.

The binding position of one phosphate group to glycerol backbone is not limited, but preferred to bind to hydroxide group at the third carbon.

The position that fatty acid or long chain alcohol is bound to glycerol backbone via ester bond is not limited, but preferred to bind to hydroxide group at the first or second carbon by ester bond.

More specifically, as phospholipids compound, geranylgeranyl 2-phosphate (GGPP), farnesyl 2-phosphate (FPP) or lysophosphatidic acid (LPA) is used. Among them, geranylgeranyl 2-phosphate (GGPP) or farnesyl 2-phosphate (FPP) is preferably used.

Therefore, the receptor protein of the present invention, its partial peptides, or salts thereof (hereinafter sometimes referred to as the receptor protein of the present invention), the DNA encoding the receptor protein of the present invention or its partial peptides (hereinafter sometimes referred to as the DNA of the present invention) and the antibodies to the receptor protein of the present invention (hereinafter sometimes referred to as the antibodies of the present invention) are specifically described for the use or applications.

### (1) Prophylactic and/or therapeutic agents for diseases associated with dysfunction of the G protein-coupled receptor protein of the present invention

The receptor protein of the present invention or the DNA encoding the receptor protein of the present invention can be used for a medicine such as a prophylactic and/or therapeutic agent for diseases associated with dysfunction of the receptor protein of the present invention.

For example, when the physiological activity of phospholipids compound as a ligand cannot be expected in a patient (deficiency of the receptor protein) due to a decrease in the receptor protein of the present invention, the activity of the ligand can be exhibited by: a) administering the receptor protein of the present invention to the patient thereby to supplement the amount of the receptor protein; or b) by increasing the amount of the receptor protein in the patient through: i) administration of the DNA encoding the receptor protein of the present invention to express the same in the patient; or ii) insertion and expression of the DNA encoding the receptor protein of the present invention in the objective cells to transplant the cells to the patient, whereby the activity of the ligand can be sufficiently exhibited. That is, the DNA encoding the receptor protein of the present invention is useful as a safe and low toxic prophylactic and/or therapeutic agent for diseases associated with dysfunction of the receptor protein of the present invention.

Specifically, the receptor protein or the DNA encoding the receptor protein of the present invention are useful for the prevention and/or treatment of central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

When the receptor protein of the present invention is used as the prophylactic/therapeutic agents described above, the receptor protein can be prepared into a pharmaceutical composition in a conventional manner.

On the other hand, where the DNA encoding the receptor protein of the present invention is used as the prophylactic/therapeutic agents described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, a) the receptor protein of the present invention or b) the DNA encoding the receptor protein can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing a) the receptor protein of the present invention or b) the DNA encoding the receptor protein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the receptor protein of the present invention varies depending on subject to be administered, organs to be administered, conditions, routes for administration, etc.; in oral administration, e.g., for the patient with hypertension, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc.; in oral administration, e.g., for the patient with hypertension, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (2) Gene diagnostic agent

By using the DNA of the present invention as a probe, an abnormality (gene abnormality) of the DNA or mRNA encoding the receptor protein of the present invention or its partial peptide in human or mammals (e.g., rats, mice, rabbits, sheep, swine, bovine, cats, dogs, monkeys, etc.) can be detected. Therefore, the DNA of the present invention is useful as a gene diagnostic agent for the damage against the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

For example, where decreased expression of the receptor protein of the present invention was detected by Northern hybridization, it can be diagnosed that there is high possibility to be a disease associated with dysfunction of the receptor protein of the present invention, or in future, to fall ill.

For example, where overexpression of the receptor protein of the present invention was detected by Northern hybridization, it can be diagnosed that there is high possibility to be a disease caused by overexpression of the receptor protein of the present invention, or in future, to fall ill.

Examples of diseases associated with dysfunction of the receptor protein of the present invention or diseases caused by overexpression of the receptor protein of the present invention include central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

### (3) A medicine containing a compound that alters an expression level of the receptor protein or its partial peptide of the present invention

By using as a probe, the DNA of the present invention can be used for screening of compounds that alter the expression level of the receptor protein or its partial peptide of the present invention.

That is, the present invention provides methods of screening compounds that alter the expression level of the receptor protein or its partial peptide of the present invention, which comprises measuring the amount of mRNA encoding the receptor protein of the present invention or its partial peptide contained in, for example, (i) a) blood, b) specific organs, c) tissues or cells isolated from the organs of non-human mammals, or in (ii) transformants, etc.

The amount of mRNA encoding the receptor protein of the present invention or its partial peptide can be specifically measured as follows.
(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., soaking stress, electric shock, light and darkness, low temperature, etc.), and the blood, specific organs (e.g., brain, liver, kidney, etc.), or tissues or cells isolated from the organs are obtained after a specified period of time.
   The mRNA encoding the receptor protein of the present invention or its partial peptide contained in the thus obtained cells is extracted from the cells, for example, in a conventional manner and quantified using, e.g., TaqManPCR, or may also be analyzed by Northern blot technique by publicly known methods.
(ii) Transformants that express the receptor protein of the present invention or its partial peptide are prepared according to the methods described above, and the mRNA encoding the receptor protein of the present invention or its partial peptide can be quantified and analyzed, as described above.

Compounds that alter the expression level of the receptor protein of the present invention or its partial peptide can be screened by the following procedures:
(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA encoding the receptor protein of the present invention or its partial peptide contained in cells are quantified and analyzed; or
(ii) Transformants are cultured in a conventional manner and a test compound is mixed in the culture medium. After a specified time (after 1 day to 7 days, preferably after 1 day to 3 days, more preferably after 2 to 3 days), the amount of mRNA in the receptor protein of the present invention or its partial peptide contained in the transformants can be quantified and analyzed.

The compounds or their salts, which are obtainable by the screening methods of the present invention, are compounds that alter the expression level of the receptor protein of the present invention or its partial peptide. Specifically, (a) compounds that potentiate the cell stimulating activities mediated by the G protein-coupled receptor (e.g., activities that promote or suppress arachidonic acid release, acetylcholine release, intracellular Ca²⁺ release, intracellular cAMP production, intracellular cGMP production, inositol phosphate production, change in cell membrane potential, phosphorylation of intracellular proteins, activation of c-fos, pH reduction, etc.) by increasing the expression level of the receptor protein of the present invention or its partial peptide ; and (b) compounds that decrease the cell-stimulating activities by reducing the expression level of the receptor protein of the present invention or its partial peptide.

The compounds include peptides, proteins, non-peptide compounds, synthetic compounds; and fermentation products. They may be novel or known compounds.

The ligand to the receptor protein of the present invention is, as described above, a phospholipids compound. Therefore, the compound that the expression level of the receptor protein or its partial peptide of the present invention, which is obtained by the above-mentioned screening method, can be used as a prophylactic and/or therapeutic agent for diseases associated with dysfunction of the receptor protein of the present invention.

Specifically, the compounds that increase the expression level of the receptor protein or its partial peptide of the present invention and potentiate the cell-stimulating activities, are useful as safe and low toxic prophylactic and/or therapeutic agent for diseases associated with dysfunction of the receptor protein of the present invention.

The compounds that decrease the expression level of the receptor protein or its partial peptide of the present invention and attenuate the cell-stimulating activities, are useful as safe and low toxic prophylactic and/or therapeutic agent for diseases caused by overexpression of the receptor protein of the present invention.

Examples of diseases associated with dysfunction of the receptor protein of the present invention or diseases caused by overexpression of the receptor protein of the present invention include central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

When the compound or its salt obtained by the screening method of the present invention is used as a pharmaceutical composition, the compound can be prepared into a pharmaceutical composition in a conventional manner.

For example, the compound can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing the compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compound or its salt varies depending on subject to be administered, organs to be administered, conditions, routes for administration, etc.; in oral administration, e.g., for the patient with hypertension, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (4) Quantification method of the ligand to the G protein-coupled receptor protein of the present invention and diagnostic method

The antibodies of the present invention are capable of specifically recognizing phospholipids compound, which is the ligand to the receptor protein of the present invention. Therefore, the antibodies can be used to quantify the phospholipids compound in a test fluid, especially for quantification by the sandwich immunoassay.

That is, the present invention provides the following quantification methods:
(i) A method of quantifying the receptor protein of the present invention in a test fluid, which comprises competitively reacting the antibody of the present invention with the test fluid and a labeled form of the receptor protein of the present invention, and measuring the ratio of the labeled receptor protein bound to the antibody; and,
(ii) A method of quantifying the receptor protein of the present invention in a test fluid, which comprises reacting the test fluid with the antibody of the present invention immobilized on a carrier and a labeled form of the other antibody of the present invention simultaneously or sequentially, and measuring the activity of the label on the immobilized carrier.

In (ii) described above, it is preferred that one antibody recognizes the N-terminal region of the receptor protein of the present invention, and the other antibody reacts with the C-terminal region of the receptor protein of the present invention.

Using monoclonal antibodies to the receptor protein of the present invention, the receptor protein of the present invention can be quantified and also detected by tissue staining or the like. For this purpose, an antibody molecule itself may be used, or F(ab')₂, Fab' or Fab fractions of the antibody molecule may also be used.

Quantification methods of the receptor protein of the present invention using antibodies of the present invention are not particularly limited. Any assay method can be used, so long as the amount of antibody corresponding to the amount of antigen (e.g., the amount of the receptor protein of the present invention) in the test fluid, the amount of antigen, or the amount of the complex between antibody and antigen can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H] and [¹⁴C] are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used include fluorescamine and fluorescein isothiocyanate. For the luminescent substance, for example, luminol, luminol derivatives, luciferin, and lucigenin are used. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, insoluble polysaccharides such as agarose, dextran, cellulose, etc.; synthetic resin such as polystyrene, polyacrylamide, silicon, etc., and glass or the like., are included.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the receptor protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the receptor protein of the present invention by the sandwich method, antibodies that bind to different sites of the receptor protein are preferably used as the monoclonal antibodies of the present invention for the primary and secondary reactions. That is, in the antibodies used for the primary and secondary reactions are, for example, when the antibody used in the secondary reaction recognizes the C-terminal region of the receptor protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody; polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying the respective immunological methods to the measurement methods of the present invention, any particular conditions or procedures are not required. Systems for measuring the receptor protein of the present invention or its salts are constructed by adding the usual technical consideration in the art to the conventional conditions and procedures. For the details of these general technical means, reference can be made to the reviews and texts.

For example, it includes Hiroshi Irie, ed. "Radioimmunoassay" (Kodansha, published in 1974), Hiroshi Irie, ed. "Sequel to the Radioimmunoassay" . (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the receptor protein of the present invention can be quantified with high sensitivity, using the antibodies of the present invention. Further, by quantifying the receptor protein of the present invention using the antibodies of the present invention, where it is detected that the concentration of the receptor protein of the present invention is reduced, it can be diagnosed that there is diseases caused by overexpression of the receptor protein of the present invention, or that there is high possibility to take ill in future.

Examples of diseases associated with dysfunction of the receptor protein of the present invention or diseases caused by overexpression of the receptor protein of the present invention include central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

### (5) Determination method of ligand to the receptor protein of the present invention other than phospholipids compound

Since intracellular Ca²⁺ release and intracellular cAMP production are observed by binding phospholipids compound to the receptor of the present invention, the receptor protein of the present invention is useful as reagents for searching and determining ligands to the receptor protein of the present invention other than phospholipids compound.

That is, the present invention provides a method for determining a ligand to the receptor protein of the present invention, which comprises bringing the cells containing the receptor protein of the present invention in contact with a test compound and measuring intracellular Ca²⁺ releasing activity (intracellular Ca2+ concentration increasing activity) or intracellular CAMP producing activity mediated by the receptor protein of the present invention.

Examples of the test compound include publicly known ligands (e.g., angiotensin, bombesin, canavinoid, cholecystokinin, glutamine, serotonin, melatonin, neuropeptide Y, opioid, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, chemokine superfamily (e.g., CXC chemokine subfamily such as IL-8, GROα, GROβ, GROγ, NAP-2, ENA-78, GCP-2, PF4, IP-10, Mig, PBSF/SDF-1, etc.; CC chemokine subfamily such as MCAF/MCP-1, MCP-2, MCP-3, MCP-4, eotaxin, RANTES, MIP-1α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc.), endothelin, enterogastrin, histamine, neurotensin, TRH, pancreatic polypeptide, galanin, lysophosphatidic acid (LPA), sphingosine 1-phosphate, and the like) as well as other substances, for example, tissue extracts and cell culture supernatants from human or mammals (e.g., mice, rats, swine, bovine, sheep, monkeys, etc.). For example, the tissue extract or cell culture supernatant is added to the receptor protein of the present invention and fractionated while assaying the cell stimulating activities, etc. to finally give a single ligand.

Specifically, the method for determining ligands of the present invention comprises determining compounds or salts thereof that have intracellular Ca²⁺ releasing activity (intracellular Ca2+ concentration increasing activity) or intracellular cAMP producing activity mediated by the receptor protein of the present invention by constructing an expression system for recombinant GPR40 and using a receptor binding assay system with the above expression system.

More specifically, the present invention provides the following features:
(i) A method for determining ligands to the receptor protein of the present invention, which comprises bringing a test compound in contact with cells containing the receptor protein of the present invention and measuring intracellular Ca²⁺ releasing activity (intracellular Ca²⁺ concentration increasing activity) or intracellular cAMP producing activity; and,
(ii) A method for determining ligands to the receptor protein of the present invention, which comprises culturing a transformant containing DNA encoding the receptor protein of the present invention, bringing a labeled test compound in contact with the receptor protein expressed on the cell membrane by said culturing, and measuring intracellular Ca²⁺ releasing activity (intracellular Ca²⁺ concentration increasing activity) or intracellular cAMP producing activity.

It is particularly preferred to confirm the binding of the test compound to the receptor protein of the present invention, followed by the tests described above.

Where cells containing the receptor protein of the present invention are used in the method of the present invention for determination of ligands, the cells may be fixed using glutaraldehyde, formalin, etc. The fixation can be made by a publicly known method.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about I to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cells containing the receptor protein and in the membrane fraction is preferably 10³ to 10⁸ molecules per cell, more preferably 10⁵ to 10⁷ molecules per cell. As the amount of expression increases, the ligand binding activity per unit of membrane fraction (specific activity) increases so that not only the highly sensitive screening system can be constructed but also large quantities of samples can be assayed with the same lot.

For carrying out the method of determining the ligand of the present invention, intracellular Ca²⁺ releasing activity (intracellular Ca2+ concentration increasing activity) or intracellular cAMP producing activity may be determined by a publicly known method, or using an assay kit commercially available. Specifically, cells containing the receptor protein of the present invention are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., Ca²⁺, cAMP, etc.) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

The kit for determining the ligand of the present invention comprises cells or cell membrane fractions containing the receptor protein of the present invention.

Since the ligand to the receptor of the present invention, which is determined as described above, regulates a physiological function of the receptor protein of the present invention by binding thereto, it can be used for a prophylactic/therapeutic agent for diseases associated with the function of the receptor protein of the present invention.

### (6) A screening method of compounds that alter the binding property between the G protein-coupled receptor protein of the present invention and the ligand (agonist, antagonist, etc.), and a medicine containing the compound that alter the binding property between the G protein-coupled receptor protein of the present invention and the ligand

By using the receptor protein of the present invention, or by constructing an expression system for the recombinant receptor protein and using a receptor binding assay system with the above expression system, a compound that alters the binding property between phospholipids compound as a ligand and the receptor protein of the present invention (e.g., peptide, protein, non-peptide compound, synthetic compound, fermentation product, etc.), or a salt thereof, can be screened efficiently.

Such compounds include (a) compounds that have the G protein-coupled receptor-mediated cell-stimulating activities (e.g., activities that promote or suppress arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention); (b) compounds that have no cell-stimulating activity (so-called antagonists to the receptor protein of the present invention); (c) compounds that potentiate the binding affinity between ligands and the G protein-coupled receptor protein of the present invention; and (d) compounds that reduce the binding affinity between ligands and the G protein-coupled receptor protein of the present invention (it is preferred to screen the compounds described in (a) using the ligand determination methods described above).

That is, the present invention provides methods of screening compounds or their salt forms that alter the binding property between ligands and the receptor protein of the present invention, its partial peptide or salts thereof, which comprises comparing (i) the case wherein the receptor protein of the present invention, its partial peptide or salts thereof are brought in contact with a ligand, with (ii) the case wherein the receptor protein of the present invention, its partial peptide or salts thereof are brought in contact with a ligand and a test compound.

The screening methods of the present invention are characterized by assaying, for example, the amount of ligand bound to the receptor protein etc., the cell-stimulating activity, etc., and comparing the property between (i) and (ii).

More specifically, the present invention provides the following screening methods:
a) A method of screening a compound or its salt that alters the binding property between a ligand and the receptor protein of the present invention, which comprises: measuring the amount of a labeled ligand bound to the receptor protein etc., when the labeled ligand is brought in contact with the receptor protein of the present invention and when the labeled ligand and a test compound are brought in contact with the receptor protein of the present invention, and, comparing the binding property between them;
b) A method of screening a compound or its salt that alters the binding property between a ligand and the receptor protein of the present invention, which comprises: measuring the amount of a labeled ligand bound to cells or the membrane fraction of the cells, when the labeled ligand is brought in contact with the cells or cell membrane fraction containing the receptor protein of the present invention and when the labeled ligand and a test compound are brought in contact with the cells or cell membrane fraction containing the receptor protein of the present invention, and, comparing the binding property between them;
c) A method of screening a compound or its salt that alters the binding property between a ligand and the receptor protein of the present invention, which comprises: measuring the amount of a labeled ligand to the receptor protein, when the labeled ligand is brought in contact with the receptor protein expressed on the cell membrane induced by culturing a transformant containing the DNA of the present invention and when the labeled ligand and a test compound are brought in contact with the receptor protein of the present invention expressed on the cell membrane induced by culturing a transformant containing the DNA of the present invention, and, comparing the binding property between them;
d) A method of screening a compound or its salt that alters the binding property between a ligand and the receptor protein of the present invention, which comprises: measuring the receptor-mediated cell-stimulating activity (e.g., the activity that promotes or suppresses arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.), when a compound (e.g., a ligand to the receptor protein of the present invention) that activates the receptor protein etc. of the present invention is brought in contact with cells containing the receptor protein of the present invention and when the compound that activates the receptor protein of the present invention and a test compound are brought in contact with cells containing the receptor protein etc. of the present invention, and, comparing the binding property between them; and,
e) A method of screening a compound or its salt that alters the binding property between a ligand and the receptor protein of the present invention, which comprises: measuring the receptor-mediated cell-stimulating activity (e.g., the activity that promotes or suppresses arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.), when a compound (e.g., a ligand for the receptor protein of the present invention) that activates the receptor protein of the present invention is brought in contact with the receptor protein of the present invention expressed on the cell membrane induced by culturing a transformant containing the DNA of the present invention and when the compound that activates the receptor protein of the present invention and a test compound are brought in contact with the receptor protein of the present invention expressed on the cell membrane induced by culturing a transformant containing the DNA of the present invention, and, comparing the binding property between them.

For ligands, phospholipids compounds such as geranylgeranyl 2-phosphate, farnesyl 2-phosphate or lysophosphatidic acid (LPA) are used.

In addition, for ligands, compounds or salts thereof that alter the binding property between phospholipids compound and the receptor protein of the present invention can be utilized. The compounds or salts thereof that alter the binding property between phospholipids compound and the receptor protein of the present invention can be obtained, for example, with a phospholipids compound as a ligand, by carrying out the screening method of the present invention described below.

Before the receptor protein of the present invention was obtained, it was required for screening G protein-coupled receptor agonists or antagonists to obtain candidate compounds first, using cells or tissues containing the G protein-coupled receptor protein or the cell membrane fraction from rats or other animals (primary screening), and then examine the candidate compounds whether the compounds actually inhibit the binding between human G protein-coupled receptor protein and ligands (secondary screening). When cells, tissues, or the cell membrane fractions were directly used, it was practically difficult to screen agonists or antagonists to the objective receptor protein, since other receptor proteins were present together.

However, using, for example, the human-derived receptor protein of the present invention, the primary screening becomes unnecessary, and compounds that inhibit the binding between ligands and the G protein-coupled receptor protein can be efficiently screened. Furthermore, it is easy to assess whether the obtained compound is an agonist or antagonist.

Hereinafter, the screening methods of the present invention are described specifically.

First, for the receptor protein of the present invention used for the screening methods of the present invention, any substance may be used so long as it contains the receptor protein of the present invention described above. The cell membrane fraction from mammalian organs containing the receptor protein of the present invention is preferred. However, it is very difficult to obtain human organs. It is thus preferable to use human-derived receptor proteins or the like, produced by large-scale expression using recombinants.

To manufacture the receptor protein of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the receptor protein of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRα promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening methods of the present invention, the material that contains the receptor protein of the present invention may be the receptor protein purified by publicly known methods, cells containing the receptor protein, or the cell membrane fraction containing the receptor protein or the like.

In the screening methods of the present invention, when cells containing the receptor protein of the present invention are used, the cells may be fixed with glutaraldehyde, formalin, etc. The cells can be fixed by publicly known methods.

The cells containing the receptor protein of the present invention are host cells that express the receptor protein. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein etc. expressed and membrane components such as cell-derived phospholipids and membrane proteins.

The amount of the receptor protein in the cells containing the receptor protein and in the membrane fraction is preferably 10³ to 10⁸ molecules per cell, more preferably 10⁵ to 10⁷ molecules per cell. As the amount of expression increases, the ligand binding activity per unit of membrane fraction (specific activity) increases so that not only the highly sensitive screening system can be constructed but also large quantities of samples can be assayed with the same lot.

To screen the compounds that alter the binding property between ligands and the receptor protein of the present invention described in a) to c), for example, an appropriate receptor protein fraction and a labeled ligand are necessary.

The receptor protein fraction is preferably a fraction of naturally occurring receptor protein or a recombinant receptor fraction having an activity equivalent to that of the natural protein. Herein, the equivalent activity is intended to mean a ligand binding activity, a signal transduction activity or the like that is equivalent to that possessed by naturally occurring receptor proteins.

For the labeled ligand, a labeled ligand and a labeled ligand analogue are used. For example, ligands labeled with [³H], [¹²⁵I], [¹⁴C], [³⁵S], etc. are used.

Specifically, to screen the compounds that alter the binding property between ligands and the receptor protein of the present invention, first, the receptor protein standard is prepared by suspending cells or cell membrane fraction containing the receptor protein of the present invention in a buffer appropriate for the screening. For the buffer, any buffer that does not interfere with the binding of ligands to the receptor protein is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80™ (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and 10⁻⁴ M - 10⁻¹⁰ M of a test compound is simultaneously added to be co-present. To examine non-specific binding (NSB), a reaction tube containing an unlabeled test compound in large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance (B₀) as 100%, when the amount of specific binding (B-NSB) is, for example, 50% or less, the test compound can be selected as a candidate substance having a potential of competitive inhibition.

To perform the methods d) and e) supra of screening the compounds that alter the binding property between ligands and the receptor protein of the present invention, the receptor protein-mediated cell-stimulating activity (e.g., activity that promotes or inhibits arachidonic acid release, acetylcholine release, intracellular Ca release, intracellular cAMP production, intracellular cGMP production, inositol phosphate production, changes in cell membrane potential, phosphorylation of intracellular proteins, activation of c-fos, pH reduction, etc.) can be measured using publicly known methods or commercially available kits.

Specifically, the cells containing the receptor protein of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

Screening by assaying the cell-stimulating activity requires cells that have expressed an appropriate receptor protein. For the cells that have expressed the receptor protein of the present invention, the cell line possessing the native receptor protein of the present invention, the cell line expressing the recombinant receptor protein described above and the like are desirable.

For the test compound, for example, peptides, proteins, non-peptide compounds, synthetic compounds, fermentation products, cell extracts, plant extracts, and animal tissue extracts are used. These compounds may be novel or known compounds.

Further, for the test compound, a compound designed for binding to the ligand binding pocket based on atom coordinate of the active site of the receptor protein of the present invention and the location of ligand binding pocket, is preferably used. The measurement of atom coordinate of the active site of the receptor protein of the present invention and the location of ligand binding pocket can be performed by publicly known methods or modified method thereof.

The kits for screening the compounds or their salts that alter the binding property between ligands and the receptor protein of the present invention comprise the receptor protein of the present invention, cells containing the receptor protein of the present invention, or the membrane fraction of cells containing the receptor protein of the present invention.

Examples of the screening kits of the present invention are as follow.
1. Reagents for screening
   a) Buffer for measurement and washing
      Hanks' balanced salt solution (Gibco) supplemented with 0.05% bovine serum albumin (Sigma).
      The solution is sterilized by filtration through a 0.45 µm filter, and stored at 4°C or may be prepared at use.
   b) Standard G protein-coupled receptor
      CHO cells expressing the receptor protein of the present invention are passaged in a 12-well plate at a density of 5 × 10⁵ cells/well followed by culturing at 37°C under 5% CO₂ and 95% air for 2 days.
   c) Labeled ligands
      Aqueous solutions of ligands labeled with commercially available [³H], [¹²⁵I], [¹⁴C], [³⁵S], etc. are stored at 4°C or -20°C, and diluted to 1 µM with the measurement buffer.
   d) Standard ligand solution

   The ligand is dissolved in and adjusted to 1 mM with PBS containing 0.1% bovine serum albumin (Sigma) and stored at -20°C.
2. Measurement method
   a) CHO cells expressing the receptor protein of the present invention are cultured in a 12-well culture plate and washed twice with 1 ml of the measurement buffer, and 490 µl of the measurement buffer is added to each well.
   b) After adding 5 µl of 10⁻³ - 10⁻¹⁰ M test compound solution, 5 µl of a labeled ligand is added to the mixture, and the cells are incubated at room temperature for an hour. To determine the amount of the non-specific binding, 5 µl of the non-labeled ligand is added in place of the test compound.
   c) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)
   d) The radioactivity is measured using a liquid scintillation counter (manufactured by Beckman Co.), and the percent maximum binding (PMB) is calculated by the equation below.
      PMB = [(B - NSB)/(B₀ - NSB)] × 100
      PMB : Percent maximum binding
      B : Value obtained in the presence of a test compound
      NSB: Non-specific binding
      B₀ : Maximum binding

The compounds or their salts, which are obtained using the screening methods or the screening kits of the present invention, are the compounds that alter the binding property between ligands and the receptor protein of the present invention. Specifically, these compounds are: (a) compounds that have the G protein-coupled receptor-mediated cell-stimulating activity (so-called agonists to the receptor protein of the present invention); (b) compounds having no cell stimulating-activity (so-called antagonists to the receptor protein of the present invention); (c) compounds that potentiate the binding affinity between ligands and the G protein-coupled receptor protein of the present invention; and (d) compounds that attenuate the binding affinity between ligands and the G protein-coupled receptor protein of the present invention.

The compounds may be peptides, proteins, non-peptide compounds, synthetic compounds, fermentation products, and may be novel or known compounds.

The compounds may also be a compound, which is designed based on atom coordinate of the active site of the receptor protein of the present invention and the location of ligand binding pocket.

Since agonists to the receptor protein of the present invention have the same physiological activities as those of phospholipids compound, which is the ligands to the receptor protein of the present invention, the agonists are useful as safe and low toxic medicines, correspondingly to the physiological activities, wherein the phospholipids compound possesses.

Since antagonists to the receptor protein of the present invention can suppress the physiological activities of phospholipids compounds, which are ligands to the receptor protein of the present invention, the antagonists are useful as safe and low toxic medicines that inhibit the physiological activities of phospholipids compound.

Specifically, the compounds or salts thereof, which are obtained by the screening methods or using the screening kits of the present invention, are useful for preventive/therapeutic agent of diseases such as central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

When the compounds or salts thereof obtained by using the screening method or the screening kit of the present invention, are used as the pharmaceutical compositions, the pharmaceutical preparations can be obtained in a conventional manner.

For example, the compounds and the ligand can be administered orally as sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive that may be mixed in tablets, capsules, etc., for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined, for example, with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

The preparations thus obtained are safe and low toxic, and can be administered to, for example, human and mammals (e.g., rats, mice, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with hypertension, the dose is normally about 0.1 mg to about 100 mg, preferably about 1.0 to about 50 mg, and more preferably about 1.0 to about 20 mg per day (as 60 kg body weight). In parenteral administration, the single dose vanes depending on subject to be administered, target organ, conditions, routes for administration, etc. but it is advantageous, e.g., for the patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (7) Medicines comprising compounds that alter the amount of the receptor protein of the present invention or its partial peptide in cell membranes

Since the antibodies of the present invention specifically recognize the receptor protein, its partial peptide, or its salt of the present invention, the antibodies can be used for screening of the compounds that alter the amount of the receptor protein of the present invention or its partial peptide in cell membranes.

That is, the present invention provides, for example, the following methods:
(i) A method of screening compounds that alter the amount of the receptor protein of the present invention or its partial peptides in cell membranes, which comprises disrupting a) blood, b) specific organs, c) tissues or cells isolated from the organs of non-human mammals, isolating the cell membrane fraction and then quantifying the receptor protein of the present invention or its partial peptide contained in the cell membrane fraction;
(ii) A method of screening compounds that alter the amount of the receptor protein of the present invention or its partial peptides in cell membranes, which comprises disrupting transformants, etc. expressing the receptor protein of the present invention or its partial peptides, isolating the cell membrane fraction, and then quantifying the receptor protein of the present invention or its partial peptides contained in the cell membrane fraction;
(iii) A method of screening compounds that alter the amount of the receptor protein of the present invention or its partial peptides in cell membranes, which comprises sectioning a) blood, b) specified organs, c) tissues or cells isolated from the organs of non-human mammals, immunostaining, and then quantifying the staining intensity of the receptor protein in the cell surface layer to confirm the protein on the cell membrane; and,
(iv) A method of screening compounds that alter the amount of the receptor protein of the present invention or its partial peptides in cell membranes, which comprises sectioning transformants, etc. expressing the receptor protein of the present invention or its partial peptides, immunostaining, and then quantifying the staining intensity of the receptor protein in the cell surface layer to confirm the protein on the cell membrane.

Specifically, the receptor protein and its partial peptides of the present invention contained in cell membrane fractions are quantified as follows.
(i) Normal or disease model non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, liver, kidney, etc.), or tissue or cells isolated from the organs are obtained after a specified period of time. The obtained organs, tissues or cells are suspended in, for example, an appropriate buffer (e.g., Tris hydrochloride buffer, phosphate buffer, Hepes buffer), and the organs, tissues, or cells are disrupted, and the cell membrane fraction is obtained using surfactants (e.g., Triton-X 100™, Tween 20™) and further using techniques such as centrifugal separation, filtration, and column fractionation.
   The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.
   The receptor protein of the present invention or its partial peptides contained in the cell membrane fraction can be quantified by, for example, the sandwich immunoassay and western blot analysis using the antibodies of the present invention.
   The sandwich immunoassay can be performed as described above, and the western blot can be performed by publicly known methods.
(ii) Transformants expressing the receptor protein of the present invention or its partial peptides are prepared following the method described above, and the receptor protein of the present invention or its partial peptides contained in the cell membrane fraction can be quantified.

The compounds that alter the amount of the receptor protein of the present invention or its partial peptides in cell membranes can be screened as follows.
(i) To normal or disease model non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the receptor protein of the present invention or its partial peptides contained in cell membranes are quantified.
(ii) Transformants are cultured in a conventional manner and a test compound is mixed in the culture medium. After a specified time (after 1 day to 7 days, preferably after 1 day to 3 days, more preferably after 2 to 3 days), the amount of the receptor protein of the present invention or its partial peptides contained in the cell membranes can be quantified.
   Specifically, the receptor protein of the present invention or its partial' peptides, which is contained in cell membrane fractions, is confirmed as follows.
(iii) Normal or disease model non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, liver, kidney, heart, pancreas, testis, etc.), or tissue or cells isolated from the organs are obtained after a specified period of time. Tissue sections are prepared from the thus obtained organs, tissues or cells in a conventional manner followed by immunostaining with the antibody of the present invention. The staining intensity of the receptor protein in the cell surface layer is quantified to confirm the protein in the cell membrane, the amount of the receptor protein of the present invention or its partial peptides in the cell membrane can be quantitatively or qualitatively confirmed.
(iv) The confirmation can also be made by the similar method, using transformants expressing the receptor protein of the present invention or its partial peptides.

The compounds or its salts, which is obtained by the screening methods of the present invention, are the compounds that alter the amount of the receptor protein or its peptide fragments of the present invention. Specifically, these compounds are; (a) compounds that potentiate the G protein-coupled receptor mediated cell-stimulating activity (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention), by increasing the amount of the receptor protein of the present invention or its partial peptides ; and (b) compounds that attenuate the cell stimulating-activity by decreasing the amount of the receptor protein of the present invention.

The compounds may be peptides, proteins, non-peptide compounds, synthetic compounds, fermentation products, and may be novel or known compounds.

The compounds that potentiate the cell-stimulating activity by increasing the amount of the receptor protein of the present invention or a partial peptide thereof in the cell membrane, are useful as safe and low toxic agent for prevention/treatment of diseases associated with dysfunction of the receptor protein of the present invention.

The compounds that attenuate the cell-stimulating activity by decreasing the amount of the receptor protein of the present invention or a partial peptide thereof in the cell membrane, are useful as safe and low toxic agent for prevention/treatment of diseases caused by overexpression of the receptor protein of the present invention.

Specifically, the compounds or salts thereof, which has a function that alters the amount of the receptor protein of the present invention or a partial peptide thereof, can be used as a preventive/therapeutic agent of diseases such as central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc.

When the compounds or salts thereof, which are obtained by using the screening method, are used as the pharmaceutical compositions, the pharmaceutical preparations can be obtained in a conventional manner.

For example, the compounds can be administered orally as sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive miscible with tablets, capsules, etc., for example, binders such as gelatin, cornstarch, tragacanth, and acacia, fillers such as crystalline cellulose, imbibers such as cornstarch, gelatin, and alginic acid, lubricants such as magnesium stearate; sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined, for example, with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

Since the preparations thus obtained are safe and low toxic, the preparations can be administered to human or mammals (e.g., rats, mice, rabbits, sheep, swine, bovine, cats, dogs, monkeys, etc.).

The dose of the compounds or their salts varies depending on subject to be administered, target organs, conditions, routes for administration, etc.; in oral administration, e.g., for the patient with hypertension, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (8) A medicine, which comprises the antibody against the receptor protein of the present invention, its partial peptide, or salts thereof

The neutralizing activity of antibodies against the receptor protein of the present invention, its partial peptides, or salts thereof refers to an activity of inactivating the signal transduction function involving the receptor protein. Thus, when the antibody has the neutralizing activity, the antibody can inactivate the signal transduction in which the receptor protein participates, for example, inactivate the receptor protein-mediated cell-stimulating activity (e.g., activity that promotes or inhibits arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.).

Therefore, the neutralizing antibodies against the receptor protein of the present invention, its partial peptides, or salts thereof can be used as a preventive/therapeutic agent of diseases such as central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc., which are caused by overexpression of the receptor protein of the present invention.

### (9) A medicine, which comprises an antisense polynucleotide of the present invention

The antisense polynucleotide of the present invention can be used as a preventive/therapeutic agent of diseases such as central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity, etc., which are caused by overexpression of the receptor protein of the present invention.

For example, where the antisense polynucleotide is used, the antisense polynucleotide itself is administered; alternatively, it is inserted into an appropriate vector such as retrovirus vector, adenovirus vector, adenovirus-associated virus vector, etc. and then administered in a conventional manner. The antisense polynucleotide may also be administered per se, or with adjuvants to assist its uptake by gene gun or through a catheter such as a catheter with a hydrogel.

Further, the antisense polynucleotide can be used as a oligonucleotide probe for diagnosis to study the presence or the expression of the DNA of the present invention in tissues or cells.

### (10) Preparation of animals, in which the DNA of the present invention is introduced

The present invention provides a non-human mammal bearing an exogenous DNA of the present invention (hereinafter merely referred to as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector bearing the exogenous DNA of the present invention or its variant DNA and capable of expressing in a mammal.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to create the DNA transgenic animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.) or rats (Wistar, SD, etc.), since they are relatively short in ontogeny and life cycle from a standpoint of creating model animals for human disease.

"Mammals" in a recombinant vector that can be expressed in the mammals include the aforementioned non-human mammals and human, etc.

The exogenous DNA of the present invention refers to the DNA of the present invention that is once isolated/extracted from mammals, not the DNA of the present invention inherently possessed by the non-human mammals. The variant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean such a DNA that expresses the abnormal receptor protein of the present invention and exemplified by the DNA that expresses a receptor protein to suppress the functions of the normal receptor protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the animal cells. For example, in the case of transfecting the human DNA of the present invention, a DNA-introduced mammal that expresses the DNA of the present invention to a high level, can be prepared by microinjecting a DNA construct (e.g., vector, etc.) ligated with the human DNA of the present invention into a fertilized egg of the target non-human mammal downstream various promoters, which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the receptor protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (1) promoters for the DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and chicken β actin promoters etc., which protein can highly express in the whole body, are preferred.

It is preferred that the vectors described above have a sequence for terminating the transcription of the desired mRNA in the DNA-introduced animal (generally called a terminator); for example, a sequence of each DNA derived from viruses and various mammals. SV40 terminator of the simian virus and the like, are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The normal translational region can be acquired as whole genomic DNA or a portion thereof from liver-, kidney-, thyroid cell-, or fibroblast cell-derived DNA of human or other mammals (e.g., rabbits, dogs, cats, guinea pigs, hamsters, rats, mice) and commercially available various genomic DNA library, or from a complement DNA as a raw material, which is prepared by publicly known methods from liver-, kidney-, thyroid cell-, or fibroblast cell-derived RNA. Alternatively, the exogenous abnormal DNA can be prepared by mutating the translational region of the normal receptor protein, which is obtained from the above cells or tissues, to variant translational region using site-directed mutagenesis.

The translational region can be prepared as a DNA construct that can be expressed in the transgenic animal by an ordinary DNA engineering method, wherein the DNA is ligated downstream the abovementioned promoters and if desired, upstream transcription termination site.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

By obtaining a homozygous animal having the transfected DNA in both of homologous chromosomes and mating a male and female of the animal, all offspring can be passaged to retain the DNA in an excess level.

In a non-human mammal bearing the normal DNA of the present invention wherein the normal DNA of the present invention has expressed to a high level, the non-human mammal promotes the function of endogenous normal DNA and sometimes may eventually develop the hyperfunction of the receptor protein of the present invention. Therefore, the animal can be utilized as a pathologic model animal for such diseases. For example, using the normal DNA transgenic animal of the present invention, it is possible to elucidate the mechanism of the hyperfunction of the receptor protein of the present invention and the pathological mechanism of the disease associated with the receptor protein of the present invention, and to determine how to treat the disease.

Since, mammals transfecting the normal DNA of the present invention exhibit a symptom of increasing a free receptor protein of the present invention, it is also possible to make use of a screening test for agents for the treatment/prevention of diseases associated with the receptor protein of the present invention.

On the other hand, non-human mammal having the exogenous abnormal DNA of the present invention can be passaged under normal breeding conditions as the DNA-bearing animal by confirming the stable retaining of the exogenous DNA via crossing. In addition, the exogenous DNA to be subjected can be utilized as a starting material by inserting the desired exogenous DNA into the plasmid described above. The DNA construct with a promoter can be prepared by conventional DNA engineering techniques. The introduction of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Since non-human mammal having the abnormal DNA of the present invention may express the abnormal DNA of the present invention at a high level, the animal may be the function inactivation type inadaptability of the receptor protein of the present invention by inhibiting the function of the endogenous normal DNA and can be utilized as its disease model animal. For example, using the abnormal DNA-introduced animal of the present invention, it is possible to elucidate the mechanism of inadaptability of the receptor protein of the present invention and to perform to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of normal receptor protein by the abnormal receptor protein of the present invention in the function inactive type inadaptability of the receptor protein of the present invention.

Further, since mammal transfected with the exogenous abnormal DNA exhibits a symptom of increasing a free receptor protein of the present invention, it can be utilize for a screening test of therapeutic medicine to the function inactivation type inadaptability of the receptor protein of the present invention.

Other potential applications of two kinds of the transgenic animals described above include:
(1) Use as a cell source for tissue culture;
(2) Elucidation of the relation to the receptor protein of the present invention that is specifically expressed or activated by the receptor protein of the present invention, by direct analysis of DNA or RNA in tissues of the DNA-introduced animal of the present invention or by analysis of the receptor protein tissues expressed by the DNA;
(3) Research in the function of cells derived from tissues that are cultured usually only with difficulty, using cells of tissue bearing the DNA cultured by a standard tissue culture technique;
(4) Screening of a drug that enhances the functions of cells using the cells described in (3) above; and,
(5) Isolation and purification of the variant receptor protein of the present invention and preparation of an antibody thereto.

Furthermore, clinical conditions of a disease associated wit the receptor protein of the present invention, including the function inactive type inadaptability of the receptor protein of the present invention can be determined using the DNA-introduced animal of the present invention. Also, pathological findings on each organ in a disease model associated with the receptor protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA-introduced animal of the present invention can serve as identification of cells capable of producing the receptor protein of the present invention, and as studies on association with apoptosis, differentiation or propagation or on the mechanism of signal transduction in these properties to inspect any abnormality therein. Thus the DNA transgenic animal of the present invention can provide an effective research material for the receptor protein of the present invention and for elucidating the function and effect thereof.

To develop a therapeutic drug for the treatment of diseases associated with the receptor protein of the present invention, including the function inactive type inadaptability of the receptor protein of the present invention, using the DNA-introduced animal of the present invention, an effective and rapid method for screening can be provided by applying the method for inspection and the method for quantification, etc. described above. It is also possible to investigate and develop a method for DNA therapy for the treatment of diseases associated with the receptor protein of the present invention, using the DNA-introduced animal of the present invention or a vector capable of expressing the exogenous DNA of the present invention.

### (11) Knockout animal

The present invention provides embryonic stem cells of the non-human mammals in which the DNA of the present invention is inactivated, and a non-human mammal in which expression of the DNA of the present invention is deficient.

The present invention provides:
(1) Non-human mammalian embryonic stem cells in which the DNA of the present invention is inactivated;
(2) The embryonic stem cells according to (1) in which the DNA of the present invention is inactivated by introducing a reporter gene (for example, β-galactosidase gene derived from Escherichia coli) into the DNA;
(3) The embryonic stem cells according to (1) as a neomycin resistant cells;
(4) The embryonic stem cells according to (1), wherein the non-human mammal is a rodent;
(5) The embryonic stem cells according to (4), wherein the rodent is a mouse;
(6) A non-human mammal deficient in expression of the DNA, wherein the DNA of the present invention is inactivated;
(7) The non-human mammal according to (6), wherein the DNA is inactivated by introducing a reporter gene (for example β-galactosidase gene derived from Escherichia coli), and the reporter gene is able to be expressed under the regulation of a promoter for the DNA of the present invention;
(8) The non-human mammal according to (6), wherein the non-human mammal is a rodent;
(9) The non-human mammal according to (8), wherein the rodent is a mouse; and
(10) A method for screening a compound or a salt thereof for promoting or inhibiting the activity of the DNA of the present invention, which is characterized by the steps of administering a test compound to the animal according to (7), and detecting expression of a reporter gene.

The non-human mammalian embryonic stem cell, in which the DNA of the invention is inactivated, means the embryonic stem cells (abbreviated as ES cells hereinafter) of the non-human mammal in which the DNA substantially has no expression ability of the receptor protein of the present invention (referred to as knockout DNA of the invention) by suppressing DNA expression ability or by substantially eliminating the activity of the receptor protein of the present invention encoded by the DNA as a result of applying an artificial mutation to the DNA of the invention involved in the non-human mammal.

The non-human mammal used is the same as described previously.

Examples of the methods for allowing the DNA of the present invention to be artificially mutated include deletion of a part of or all the DNA sequence, and insertion or substitution of other DNAs by gene engineering. The knockout DNA of the present invention may be prepared by displacing reading frames of codon, or by destroying the functions of promoters or exons.

The non-human mammalian embryonic stem cells in which the DNA of the present invention is inactivated (abbreviated as DNA inactivated ES cells of the present invention or knockout ES cell of the present invention hereinafter) can be obtained, for example, by the steps comprising isolating the DNA of the present invention contained in the desired non-human mammal; disabling synthesis of perfect mRNAs by destroying the function of exons by inserting a drug resistant gene represented by a neomycin resistant gene and hygromycin resistant gene, or a reporter gene represented by a lacZ (β-galactosidase gene); cat (chloramphenicol acetyltransferase gene) into exon portions, or by inserting a DNA sequence (for example a poly-A addition signal) for terminating translation of the gene into the intron portion between the exons; introducing the DNA chain constructed so as to destroy the gene as a result of insertion above (abbreviated as a targeting vector hereinafter) into the chromosome of the animal by a homologous recombination method; analyzing the ES cells obtained by a Southern hybridization analysis using the DNA sequence on or in the vicinity of the DNA of the invention as a probe, or by a PCR method using the DNA sequence on the targeting vector and the DNA sequence in the vicinity of a region except the DNA of the invention used for preparing the targeting vector as primers; and selecting the knockout ES cells of the invention.

Established cell lines as described above may be used as the original ES cells before inactivating the DNA of the present invention by a homologous recombination method, or new cell lines may be established according to the Evans and Kaufman method known in the art. For example, the 129 line ES cells are usually used today as the mouse ES cells. However, since the immunological background is not clear in the 129 ES cell line, cell lines established using BDF₁ (F₁ of C57BL/6 and DBA/2) mouse, in which a small level of ovulation of (C57BL/6 mouse and C57BL/6) is improved by cross-breeding with DBA/2 mouse, may be favorably used in place of the 129 ES cell line for the purpose of obtaining ES cells from a pure line whose genetic background is immunologically clear. The BDF1 mouse is advantageous due to its high level of ovulation in addition to toughness of eggs. Moreover, since the BDF1 mouse is a descendant of the C57BL/6 mouse, the genetic background the ES cells obtained using the BDF1 mouse may be traced to that of the C57ML/6 mouse by back cross-breeding with the C57BL/6 mouse when a disease model mouse is created using the ES cells.

While blastocysts 3.5 days after fertilization have been usually used for establishing the ES cells, many initial stage embryos may be efficiently obtained by collecting 8-cell stage embryos and cultivating to the blastocysts.

While either male or female ES cells may be used, the male ES cells are usually advantageous for preparing regenerative chimera lines. It is desirable to discriminate the ES cells to be female or male as soon as possible in order to save the labor of cultivation.

An example of the method for discriminating male or female of the ES cells is to amplify and detect the gene at the sex determining region on the Y-chromosome by PCR. Since the number of the ES cells in only one colony (about 50 cells) is sufficient for the karyotype analysis in contrast to the conventional method requiring about 10⁶ cells, discrimination of male or female becomes possible at the primary selection of the ES cells at the early stage of cultivation. Selection of male cells at the early stage permits the labor of initial cultivation to be largely reduced.

The number of the chromosomes can be confirmed by a G-banding method in the secondary selection. While the number of the chromosomes of the ES cell is preferably accounts for 100% of the number of the chromosomes in the normal cell, the ES cells are desirably cloned again to normal cells (for example, the number of the chromosomes is 2n = 40 in mouse), when it is difficult to obtain the normal ES cells in relation to physical operations for establishing the cell line.

Although proliferating ability of the embryonic stem cell line obtained as described above is usually very good, sub-culture should be carefully performed since regenerative ability tends to be lost. For example, the cells are cultivated on appropriate feeder cells such as STO fibroblast cells in the presence of LIF (1 to 10,000 U/ml) in a carbon dioxide incubator (preferably about 5% of carbon dioxide and about 95% of air, or about 5% of carbon dioxide and about 90% of air) at 37°C. For sub-cultivation, the cells are divided into single cells by, for example, a trypsin treatment (usually about 0.001 to 0.5% trypsin/ about 0.1 to 5 mM EDTA, preferably about 0.1% trypsin/ about 1 mM EDTA), and seeded on the freshly prepared feeder cells. While such sub-cultivation is performed for every 1 to 3 days, the cells are observed at every sub-cultivation to abandon the cells having abnormal configurations.

The ES cells are able to differentiate into various cell types such as pariental cells, internal organ cells and heart muscle cells by monolayer cultivation up to high density, or by floating cultivation until cell clots are formed under an appropriate condition (M. J. Evans and M. H. Kaufman, Nature vol. 292, p154, 1981; G. R. Martin, Proceeding of National Academy of Science U.S.A, vol. 78, p7634, 1981; T. C. Doetshman et. al., Journal of Embryology and Experimental Morphology, vol. 87, p27, 1985). The DNA expression deficient cells of the present invention obtained by differentiation of the ES cells of the present invention is useful for in vitro cell biological investigations of the polypeptide of the present invention.

The DNA expression deficient non-human mammal of the present invention can be discriminated from normal animals by indirectly comparing the number of expression using the number of mRNAs of the animal by a conventional method.

The same non-human mammals as described above may be used.

The DNA of the present invention can be knocked out in the DNA expression deficient non-human mammal of the invention by introducing the targeting vector prepared as described above into the mouse embryonic cells or mouse egg cells, and substituting the DNA of the invention on the chromosomes of the mouse embryonic stem cells or mouse egg cells with the DNA sequence in which the DNA of the invention is inactivated by introducing the targeting vector by homologous recombination of the gene.

The cells in which the DNA of the present invention is knocked out can be judged by a Southern hybridization analysis using the DNA of the invention or the DNA sequence in the vicinity thereof as a probe, or by a PCR method using as primers the DNA sequence on the targeting vector and the DNA sequence in the neighbor region other than the DNA of the present invention originating from the mouse used for the targeting vector. When the embryonic stem cells of the non-human mammal are used, the cell line in which the DNA of the invention is inactivated is cloned by homologous recombination of genes, the cells are injected into the embryos or blastocysts of the non-human mammal at eight cell stage, and chimera stem cells prepared are transplanted into the uterus of the non-human mammal in spurious pregnancy. The prepared animal is a chimera animal comprising the cells having loci of the normal DNA of the present invention and loci of the artificially mutated DNA of the present invention.

When the chimera animal has the DNA loci of the present invention in which a part of germ cells has been mutated, individuals, in which all the tissues are artificially mutated, comprising the cells having the DNA loci of the present invention are obtained by selecting from a population obtained by cross-breeding of the chimera animal with the normal animal by, for example, coat color judgment. The individuals obtained as described above are usually deficient in hetero-expression of the polypeptide of the invention, and the individuals deficient in hetero-expression of the polypeptide of the invention are cross-bred with each other to obtain individuals deficient in homo-expression of the polypeptide of the invention from babies thereof.

When egg cells are used, a transgenic non-human mammal having the targeting vector introduced into the chromosome can be obtained by injecting the DNA solution into the nucleus of the egg cell by, for example, a microinjection method, and individuals mutated in the loci of the DNA of the present invention by homologous recombination of genes are selected by comparing with the transgenic non-human mammal.

The individual in which the DNA of the present invention is knocked out can be sub-bred in a usual breeding environment after confirming that the DNAs of the individual of the animal obtained by cross-breeding are also knocked out.

Acquisition and maintenance of the genital line may be carried out by a conventional method. Homozygote animals having the inactivated DNA in both homologous chromosomes can be obtained by cross-breeding of a male and female having the inactivated DNA. The homozygote animal can be efficiently obtained by breeding so that the mother animal gives rise to babies in a proportion of one normal individual and plural homozygotes. The homozygote and heterozygote having the inactivated DNA are sub-bred by cross-breeding of the heterozygous animals.

The non-human mammal in which the DNA of the present invention is inactivated is quite useful for preparing the non-human mammal in which the DNA of the present invention is deficient.

Since the non-human mammal in which the DNA of the present invention is inactivated is deficient in various biological activities induced from the receptor protein of the present invention, the animal serves as a disease model caused by inactivation of the biological activity of the receptor protein of the present invention. Accordingly, the mammal is useful for elucidation of the cause of these diseases and for therapy of these diseases.

### (11a) Screening method of compounds having therapeutic and preventive effects against diseases caused by deletion and damage of the DNA of the present invention

The DNA expression deficient non-human mammal of the present invention can be used for screening of compounds having therapeutic and preventive effects against the diseases caused by deletion or damage of the DNA of the present invention.

That is, the present invention provides a screening method of compounds or salts thereof having therapeutic and preventive effects against the diseases caused by deletion or damage of the DNA of the present invention, wherein the test compounds are administered to the DNA expression deficient non-human mammals of the present invention, and changes in the mammal are observed and measured.

Examples of the DNA expression deficient non-human mammals of the present invention used in the screening method are the same as those described above.

Examples of the test compound include peptides, proteins, non-peptide compounds, synthetic compounds, fermentation products, cell extracts, plant extracts, animal tissue extracts and plasma. These compounds may be novel compounds, or publicly known compounds.

Specifically, the therapeutic and preventive effects of the test compound can be tested by comparing changes of the animal in organs, tissues and symptoms of diseases as indices with results obtained from non-treated control animals after treating the DNA expression deficient non-human mammal of the present invention with the test compound.

The methods for treating the test animal with the test compound include oral administration and intravenous injection, and the methods may be appropriately selected depending on the symptoms of the test animal and properties of the test compound. The dosage of the test compound may be also appropriately selected depending on the administration method and the properties of the test compound.

For the screening method, where the test compound is administered to the test animal, the test compound, by which blood sugar level of the test animal is lowered at least 10%, preferably at least 30%, more preferably at least 50%, can be selected as a compound having the therapeutic and preventive effects against the above-mentioned diseases.

The compound obtained by the screening method of the present invention is selected from the test compounds described above. Since the compound has therapeutic and preventive effects against diseases caused by deletion and damage of the receptor protein of the present invention (e.g., central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity), the compound can be used as a safe and low toxic medicine such as a therapeutic and preventive agent. The compounds derived from the compounds obtained by screening may be used as well.

The compounds obtained by the screening method above may form salts, which are salts with physiologically acceptable acids (such as inorganic and organic acids) and bases (shuch as alkali metal), and physiologically acceptable acid addition salts are preferable among them. Examples of the acids for forming the salts include inorganic acids (such as hydrochloric acid, phosphoric acid, hydrobromic acid and sulfuric acid) and organic acids (acetic acid, formic acid, propionic acid, fumaric acid, maleic acid, succinic acid, tartaric acid, citric acid, malic acid, oxalic acid, benzoic acid, methanesulfonic acid and benzenesulfonic acid).

The medicine containing the compound and the salt thereof obtained by the screening method can be manufactured by the same method as producing the medicine containing the compound that alters the binding property between the aforementioned receptor protein of the present invention and the ligand.

Since the formulation obtained as described above is safe and has low toxicity, it can be administered to mammals (such as rat, mouse, guinea pig, sheep, rabbit, swine, bovine, horse, cat, dog and monkey).

While the dosage of the compound and salts thereof differs depending on the disease, administration object and administration route, the dosage is about 0.1 to 100 mg, preferably about 1.0 to 50 mg, and more preferably about 1.0 to 20 mg a day, when the compound is orally administered to the patient with hypertension (body weight 60 kg). While the dosage of non-oral administration also differs depending on the administration object and disease, the dosage is about 0.01 to 30 mg, preferably about 0.1 to 20 mg, and more preferably about 0.1 to 10 mg a day, when the compound is administered by intravenous injection as an injection agent to the patient with hypertension (body weight 60 kg). A dosage converted into a body weight of 60 kg may be administered for other mammals.

### (11b) Screening method of compounds that enhance or inhibit the activity of promoter for the DNA of the present invention

The present invention provides a method for screening a compound or a salt thereof that enhances or inhibits the activity of the promoter for the DNA of the present invention, wherein the test compound is administered to the DNA expression deficient non-human mammal of the present invention to detect expression of the reporter gene.

In the screening method above, examples of the DNA expression deficient non-human mammal of the present invention include mammals, wherein the DNA of the present invention is inactivated in the DNA expression deficient non-human mammal of the invention by introduction of the reporter gene, and the reporter gene is expressed under the control of the promoter for the DNA of the present invention.

Examples of the test compound are the same as those described above.

The reporter genes are also the same as described above, and β-galactosidase gene (lacZ), soluble alkaline phosphatase gene or luciferase gene is favorably used.

Since the reporter gene is under the control of the promoter for the DNA of the present invention in the DNA expression deficient mammal of the present invention in which the DNA of the present invention is substituted with the reporter gene, the activity of the promoter can be detected by tracing expression of a substance encoded by the reporter gene.

For example, when a part of the DNA region encoding the receptor protein of the present invention is substituted with β-galactosidase gene (lacZ) derived from Escherichia coli, β-galactosidase is expressed in place of the receptor protein of the present invention in the tissue where the receptor protein of the present invention has been intrinsically expressed. Accordingly, expression of the receptor protein of the present invention in an animal can be readily observed by staining the tissue using a substrate of β-galactosidase such as a 5-bromo-4-chloro-3-indolyl-β-galactopyranoside (X-gal). Specifically, a mouse deficient in the receptor protein of the present invention, or a slice of the tissue thereof is fixed with glutaraldehyde, the sample is stained with a staining solution containing X-gal at room temperature or at around 37°C for 30 minutes to 1 hour after washing with a phosphate buffered saline (PBS), the tissue sample is washed with 1 mM EDTA/PBS to stop the β-galactosidase reaction, and the stained sample is observed. The mRNA encoded by lacZ may be detected by a conventional method.

The compound or the salt thereof obtained by the screening method above is selected from the test compounds above, and it is the compound that enhances or inhibits the activity of the promoter for the DNA of the present invention.

The compounds obtained by the screening method above may form salts, and salts with physiologically acceptable (such as inorganic acids) and bases (such as organic acids) are used. Among them, physiologically acceptable acid addition salts are preferable. Examples of the acids for forming the salts include inorganic acids (such as hydrochloric acid, phosphoric acid, hydrobromic acid and sulfuric acid), and organic acids (such as acetic acid, formic acid, propionic acid, fumaric acid, maleic acid, succinic acid, tartaric acid, citric acid, malic acid, oxalic acid, benzoic acid, methanesulfonic acid and benzenesulfonic acids).

Since the compound for enhancing the activity of the promoter for the DNA of the present invention can promote expression of the receptor protein of the present invention to promote the function of the receptor protein, it is useful as a medicine such as a therapeutic and preventive agent for the diseases associated with dysfunction of the receptor protein of the present invention.

Since the compound for inhibiting the activity of the promoter for the DNA of the present invention can inhibit expression of the receptor protein of the present invention to inhibit the function of the receptor protein, it is useful as a medicine such as a therapeutic and preventive agent for the diseases associated with overexpression of the receptor protein of the present invention.

Specifically, the compound or its salt that enhances or inhibits the promoter activity for the DNA of the present invention can be used as a prophylactic and therapeutic agent for diseases such as central dysfunction (e.g., Alzheimer's disease, senile dementia, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.), cancer (e.g., non-small cell lung carcinoma, cancer of ovary, prostate cancer, stomach cancer, bladder cancer, breast cancer, uterocervical cancer, colon cancer, rectum cancer, etc.), diabetes mellitus, immune system disorders (e.g., autoimmune diseases, AIDS, atopic dermatitis, allergic diseases, immunodeficiency, asthma, rheumatoid arthritis, psoriasis, arteriosclerosis, diabetes mellitus, Alzheimer's disease, etc.), diseases relating to liver and gallbladder (cirrhosis, hepatitis, hepatic dysfunction, cholestasis, calculi, etc.), alimentary diseases (ulcer, enterisis, dyspepsia, irritable colitis, ulcerative colitis, diarrhea, ileus, etc.), angst, pain, obesity

The compounds induced from the compounds obtained by the screening method above are also used as described above.

The medicine containing the compound and the salt thereof obtained by the screening method above can be manufactured by the same method as producing the medicine containing the compound that alters the binding property between the receptor protein of the present invention or a salt.thereof, and the ligand.

Since the formulation obtained as described above is safe and has low toxicity, it can be administered to human or mammals (e.g., rat, mouse, guinea pig, rabbit, sheep, swine, bovine, horse, cat, dog and monkey).

While the dosage of the compound and salts thereof differs depending on the disease, administration object and administration route, the dosage is about 0.1 to 100 mg, preferably about 1.0 to 50 mg, and more preferably about 1.0 to 20 mg a day for a patient with hypertension (body weight 60 kg) when the compound for promoting the activity of the promoter against the DNA of the invention is orally administered for therapy of the respiratory organ diseases. While the dosage of non-oral administration also differs depending on the administration object and disease, the dosage is about 0.01 to 30 mg, preferably about 0.1 to 20 mg, and more preferably about 0.1 to 10 mg a day for a patient with hypertension (body weight 60 kg) when the compound is administered by intravenous injection as an injection agent for therapy of the respiratory organ diseases. A dosage converted into a body weight of 60 kg may be administered for other mammals.

The DNA expression deficient non-human mammal of the invention is quite useful for screening the compound for enhancing or inhibiting the activity of the promoter for the DNA of the present invention, and is able to largely contribute to elucidation of causes of various diseases ascribed to deficiency of expression of the DNA of the present invention and to the development of the pharmaceutical product for therapy and prevention.

Further, where by using the DNA containing the promoter region for the receptor protein of the present invention, ligating genes encoding various proteins downstream thereto, and thereafter injecting the DNA to oocyte of animal, the so-called transgenic animal (gene transferred animal) is prepared, it becomes possible to investigate the in vivo function of the receptor protein by synthesizing it specifically. Furthermore, where an appropriate reporter gene is ligated to the above-mentioned promoter region and cell lines, in which the reporter gene can be expressed, are established, they can be used as a screening system for low molecular weight compound, which has function specifically enhancing or inhibiting the producing ability of the receptor protein of the present invention in vivo.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA:: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid
- * :: corresponding to stop codon
- Me :: methyl
- Et :: ethyl
- Bu :: butyl
- Ph :: phenyl
- TC :: thiazolidine-4(R)-carboxamide

The substituents, protective groups and reagents, which are frequently used throughout the specification, are shown by the following abbreviations.
- Tos :: p-toluenesulfonyl
- CHO :: formyl
- Bzl :: benzyl
- Cl₂Bzl :: 2,6-dichlorobenzyl
- Bom :: benzyloxymethyl
- Z :: benzyloxycarbonyl
- Cl-Z :: 2-chlorobenzyloxycarbonyl
- Br-Z :: 2-bromobenzyloxycarbonyl
- Boc :: t-butoxycarbonyl
- DNP :: dinitrophenol
- Trt :: trityl
- Bum :: t-butoxymethyl
- Fmoc :: N-9-fluorenylmethoxycarbonyl
- HOBt :: 1-hydroxybenztriazole
- HOOBt:: 3,4-dihydro-3-hydroxy-4-oxo-1,2,3-benzotriazine
- HONB :: 1-hydroxy-5-norbornene-2,3-dicarboximide
- DCC :: N,N'-dicyclohexylcarbodiimide
- Fam :: 6-carboxy-fluorescein
- Tamra :: 6-carboxy-tetramethyl-rhodamine

The sequence identification numbers in the sequence listing of the specification indicates the following sequence, respectively.

### SEQ ID NO: 1

This shows the amino acid sequence of TGR4, the human-derived novel G protein-coupled receptor protein of the present invention.

### SEQ ID NO: 2

This shows the base sequence of cDNA encoding TGR4, the human-derived novel G protein-coupled receptor protein of the present invention.

### SEQ ID NO: 3

This shows the base sequence of primer 1 used in the PCR reaction of Reference Example 1 described below.

### SEQ ID NO: 4

This shows the base sequence of primer 2 used in the PCR reaction of Reference Example 1 described below.

### SEQ ID NO: 5

This shows the base sequence of probe used in the PCR reaction of Reference Example 2 described below.

### SEQ ID NO: 6

This shows the base sequence of primer used in the PCR reaction of Reference Example 2 described below.

### SEQ ID NO: 7

This shows the base sequence of primer used in the PCR reaction of Reference Example 2 described below.

### SEQ ID NO: 8

This shows the amino acid sequence of TGR4, the rat-derived novel G protein-coupled receptor protein of the present invention.

### SEQ ID NO: 9

This shows the base sequence of cDNA encoding TGR4, the rat-derived novel G protein-coupled receptor protein of the present invention.

### SEQ ID NO: 10

This shows the amino acid sequence of TGR4, the mouse-derived novel G protein-coupled receptor protein of the present invention.

### SEQ ID NO: 11

This shows the base sequence of cDNA encoding TGR4, the mouse-derived novel G protein-coupled receptor protein of the present invention.

### SEQ ID NO: 12

This shows the base sequence of primer used in the PCR reaction of Example 4 described below.

### SEQ ID NO: 13

This shows the base sequence of primer used in the PCR reaction of Example 4 described below.

### SEQ ID NO: 14

This shows the base sequence of primer used in the PCR reaction of Example 5 described below.

### SEQ ID NO: 15

This shows the base sequence of primer used in the PCR reaction of Example 5 described below.

### SEQ ID NO: 16

This shows the base sequence of primer used in the PCR reaction of Example 8 described below.

### SEQ ID NO: 17

This shows the base sequence of primer used in the PCR reaction of Example 8 described below.

### SEQ ID NO: 18

This shows the base sequence of probe used in the PCR reaction of Example 8 described below.

The transformant Escherichia coli DH5α/pCR-Blunt-TGR4 obtained in Example 1 described below was on deposit with International Patent Organisms Depository, National Institute of Advanced Industrial Science and Technology (formerly, National Institute of Bioscience and Human-Technology (NIBH), Ministry of International Trade and Industry), located at Central 6, 1-1-1 Higashi, Tsukuba, Ibaraki, as the Accession Number FERM BP-7115 on April 3, 2000 and with Institute for Fermentation (IFO), located at 2-17-85 Juso-honmachi, Yodogawa-ku, Osaka-shi, Osaka, as the Accession Number IFO 16409 on March 23, 2000.

The transformant Escherichia coli JM109/pTArTGR4 obtained in Example 4 described below was on deposit with International Patent Organisms Depository, National Institute of Advanced Industrial Science and Technology, located at Central 6, 1-1-1 Higashi, Tsukuba, Ibaraki, as the Accession Number FERM BP-8147 on August 8, 2002.

- The transformant Escherichia coli JM109/pTAmTGR4 obtained in Example 5 described below was on deposit with International Patent Organisms Depository, National Institute of Advanced Industrial Science and Technology, located at Central 6, 1-1-1 Higashi, Tsukuba, Ibaraki, as the Accession Number FERM BP-8146 on August 8, 2002.

### EXAMPLES

The present invention is described in detail below with reference to Reference Examples and Examples, but is not deemed to limit the scope of the present invention thereto. The gene manipulation procedures using Escherichia coli were performed according to the methods described in the Molecular Cloning.

### REFERENCE EXAMPLE 1

### Cloning of the cDNA encoding TGR4, the G protein-coupled receptor protein and determination of the base sequence

Using human brain-derived cDNA (GIBCO BRL) as a template and two primers, namely, primer 1 (5'-GGG TCG ACA TGT TAG CCA ACA GCT CCT CAA CCA AC-3'; SEQ ID NO: 3) and primer 2 (5'-GGA CTA GTT CAG AGG GCG GAA TCC TGG GGA CAC-3'; SEQ ID NO: 4), PCR was carried out. The reaction solution in the above reaction comprised of 1/50 volume of the above-mentioned cDNA as a template, 1/50 volume of Pfu Turbo DNA Polymerase (STRATAGENE), 0.8 µM each of primer 1 and primer 2, 400 µM of dNTPs, and a buffer attached to the enzyme to make the total volume 50 µl. The PCR reaction was carried out by reaction of 94°C for 2 minutes, then a cycle set to include 94°C for 15 seconds followed by 56°C for 15 seconds and 72°C for 100 seconds, which was repeated 40 times, and finally, extension reaction at 72°C for 5 minutes. The PCR product was subcloned to plasmid vector pCR-Blunt Vector (Invitrogen) following the instructions attached to the Zero Blunt TOPO PCR Cloning Kit (Invitrogen). The plasmid was then introduced into Escherichia coli DH5α, and the clones harboring the interest cDNA were selected on LB agar plates containing kanamycin. As a result of analysis for sequence of each clone, cDNA sequence encoding the novel G protein-coupled receptor protein, which consists of 1116 base pairs, was obtained (SEQ ID NO: 2). The novel G protein-coupled receptor protein having the amino acid sequence deduced from the cDNA was designated TGR4, and the transformant containing the DNA represented by SEQ ID NO: 2 was designated Escherichia coli DH5α/pCR-Blunt-TGR4.

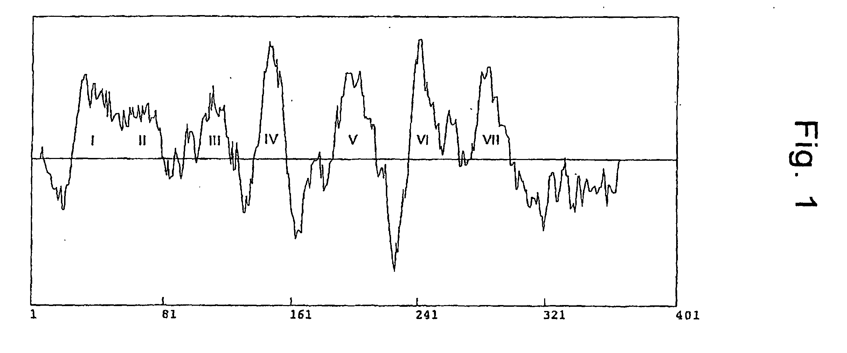

The hydrophobicity plot of TGR4 is shown in FIG 1.

### REFERENCE EXAMPLE 2

### Analysis of expression distribution of TGR4 in tissues

Analysis of expression distribution of TGR4, the G protein-coupled receptor protein in tissues was performed by semi-quantitative PCR method (TaqMan method) using real-time monitoring. The TaqMan method is based on the principle, wherein specific PCR chains amplified by PCR can be detected and quantified in real-time by fluorescent intensity of the fluorescent probe called TaqMan probe using SDS7700.

A probe and primers, which specifically recognize TGR4, were designed by using Primer Express (software supplied by PE Applied Biosystems) and synthesized.

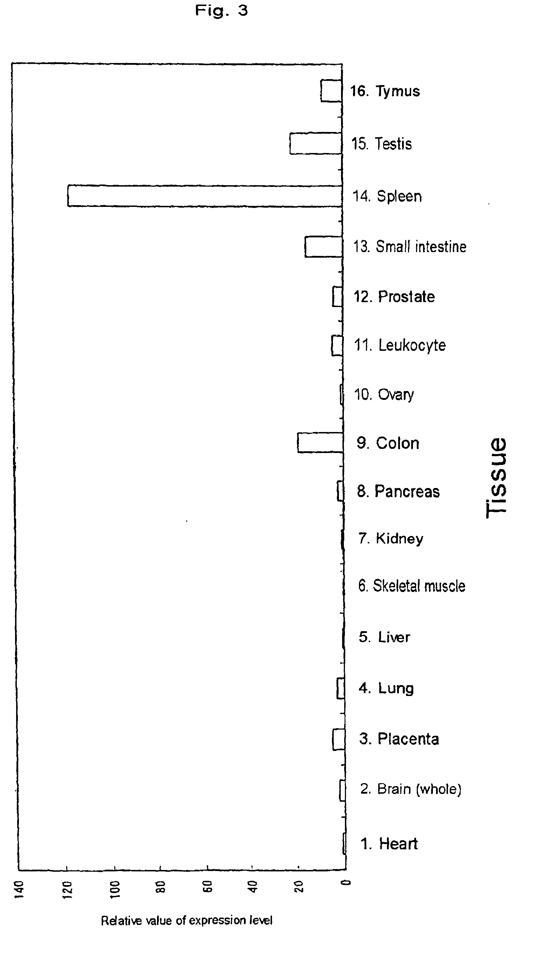

The reaction solution in the TaqMan PCR reaction comprised of 16 cDNAs of Human MTC Panel I & II (CLONTECH) as a template, 12.5 µl of 2x TaqMan Universal PCR Master Mix (PE Applied Biosystems), 200 nM TaqMan probe (SEQ ID NO: 5), and 400 nM each of TaqMan primers (SEQ ID NO: 6 and SEQ ID NO: 7) to make the total volume 25 µl. The PCR reaction was carried out by reaction of 50°C for 2 minutes and 95°C for 10 minutes, then a cycle set to include 95°C for 15 seconds followed by 62°C for 1 minute. Just as the completion of the reaction, automatic quantitative analysis for PCR was performed. Standardization was done with the similar system using TaqMan GAPDH Control Reagent (PE Applied Biosystems). The result is shown in FIG. 3.

### EXAMPLE 1

Assay for an expression level of reporter gene with phospholipids compound in the CHO cells, in which TGR4 is transiently expressed

The expression vector plasmid pAK-TGR4 or. pAK-Gi, which was prepared by inserting TGR4 cDNA or suppressive G protein α subunit Gi cDNA into pAKKO-111H, the expression plasmid vector for animal cells (the same plasmid vector as pAKKO-1.11H described in Biochem. Biophys. Acta by Hinuma, S. et al., Vol. 1219, pp. 251-259, 1994), and original pAKKO-111H, in which no specific gene was inserted, were transfected into the CHO cells by the following method.

Using these vectors, Escherichia coli JM109 was transformed. A colony obtained was isolated and cultured for preparation of plasmid with QIAGEN Plasmid Maxi Kit (Qiagen). Further, plasmid of pCRE-Luc (CLONTECH), in which luciferase gene is ligated as a reporter downstream cAMP response element (CRE), was prepared in a similar method. The CHO-mock cells, wherein the CHO (dhfr⁻) cells were transformed with pAKKO-111H, were seeded in 96-well black plate (Costar) at 40,000 cells/well and 100 µl of culture volume, and were cultured for overnight. For a culture on the plate, DMEM (Dulbecco's modified Eagle's medium, CibcoBRL) supplemented with nothing but 10% fetal bovine serum was used. In order to detect an increase of expression level of the reporter gene more clearly, the pAK-Gi was co-transfected with the pAK-TGR4. Each plasmid was added to 240 µl of Opti-MEM-I (GibcoBRL) at the ratio of 7:3:1 of pAK-TGR4:pAK-Gi:reporter plasmid pCRE-Luc. This solution was mixed with the same volume of solution wherein 10 µl of Lipofectamine 2000 was added to 240 µl of Opti-MEM-I to form a complex of liposome and plasmid according to the method described in the manual attached to Lipofectamine 2000. To culture of CHO-mock cells, 25 µl/well of the above-mentioned solution was added. After 4 hours at 37°C, a culture broth was replaced to an assay buffer (DMEM supplemented with 0.1% bovine serum albumin) to be serum-free for introduction of the plasmid. Then, these cells were cultured at 37°C under the condition of 5% CO₂ for overnight.

To the CHO-mock cells transfected as described above, geranylgeranyl 2-phosphate (GGPP, Sigma), farnesyl 2-phosphate (FPP, Sigma) and lysophosphatidic acid (LPA, Sigma) were added to the concentration of 1 µM, 0.1 µM, and 0.01µM, respectively, and incubated at 37°C under the condition of 5% CO₂ for 4 hours. After discarding the supernatant, 50 µl of Picagene LT2.0, the substrate for assaying luciferase activity were added, and luminescence level of luciferase was measured using plate reader (ARVO sx multilabel counter, Wallac).

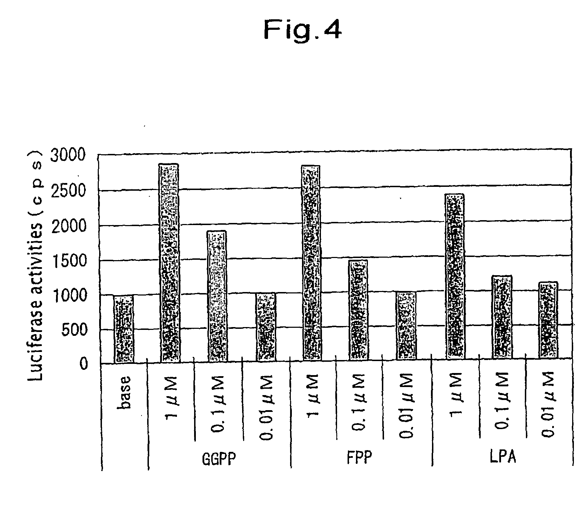

As a result, in the cells wherein the TGR4 gene was introduced, increase of luciferase activity was detected by GGPP and FPP at the concentration of 1 µM and 0.1 µM, and 1 µM LPA (FIG 4). From this result, it was indicated that the TGR4-expressing CHO cells are activated by the above-mentioned phospholipids compound.

### EXAMPLE 2

### Assay for an intracellular calcium ion mobilization activity in the TGR4-expressing CHO cells by phospholipids compound

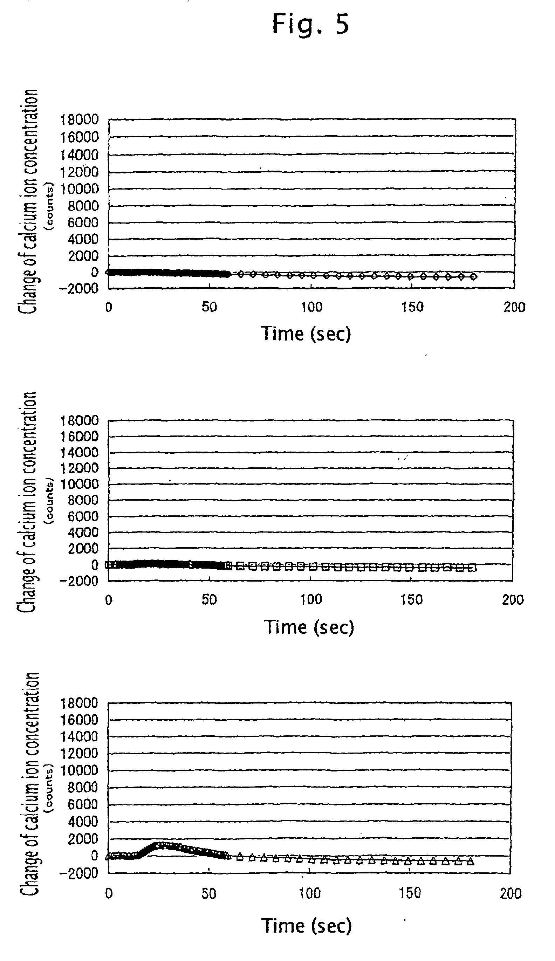

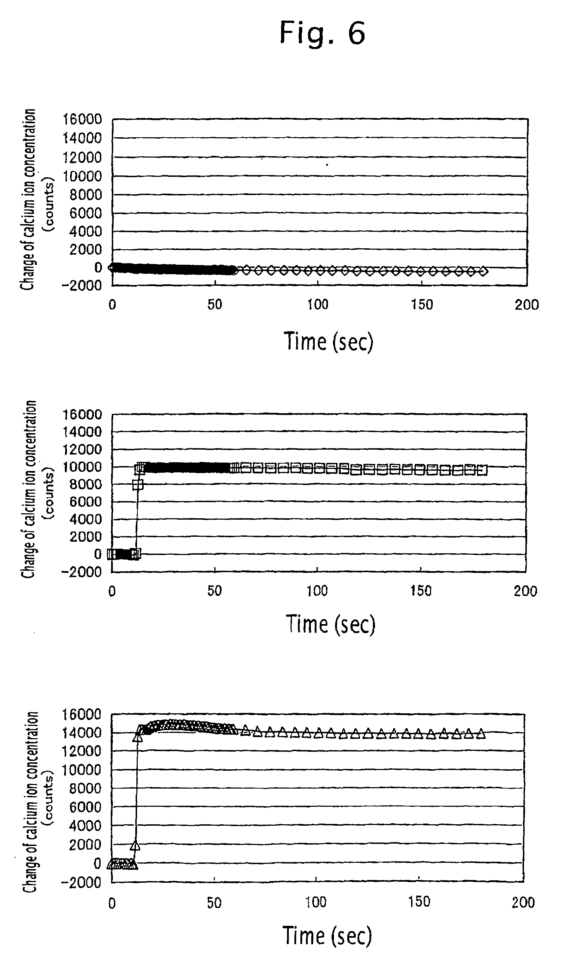

Using TGR4 expression vector prepared in EXAMPLE 1, according to the publicly known method, the CHO cells stably expressing TGR4, which is designated CHO-TGR4, were prepared. CHO-TGR4 or CHO-mock cells, in which no receptor gene is introduced, was seeded to 96-well plate of Black plate, Clear bottom available from Costar at the concentration of 30,000 cells/well. After cultivation at 37°C in the presence of 5% CO₂ for overnight, the cells were used for assay. For washing buffer, Hank's balanced salt solution (HBSS, GibcoBRL) supplemented with 20 mM HEPES (pH7.4) and 2.5 mM probeneside (Sigma) was prepared. For sample buffer, the washing buffer supplemented with 0.1% bovine serum albumin (BSA, Sigma) was used. The washing buffer supplemented with 1% FBS, 4 µM Fluo 3-AM (Wako Pure Chemicals) and 0.04% Pluronic acid (Molecular Probes) was used as a dye loading solution. After discarding the medium, adding the dye loading solution and incubating the cells at 37°C for 1 hour, using the plate washer, the cells were washed with the washing buffer. The cells were set to Fluorometric Imaging Plate Reader (FLIPR, Molecular Device) and the change of intracellular calcium ion concentration during three minutes after addition of sample was measured. FPP and LPA were added to sample to make a final concentration 2.5 µM each. As shown in FIGs 5 and 6, it was found that where FPP and LPA were added, intracellular calcium ion mobilization activity was increased in the manner specific to CHO-TGR4.

### EXAMPLE 3

### Assay for cAMP production level in the TGR4 expressing CHO cells by phospholipids compound

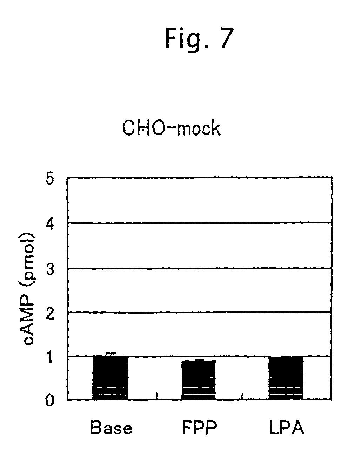

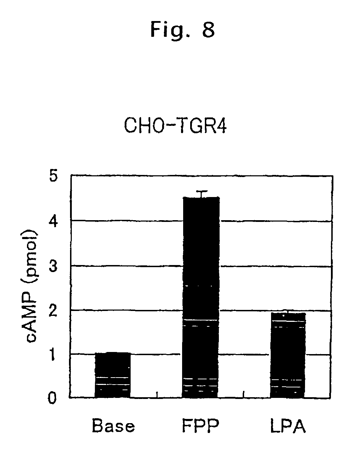

The CHO-TGR4 prepared in EXAMPLE 2, or the CHO-mock cells, in which no receptor gene is introduced, were seeded on 96-well plate at the concentration of 25,000 cells/well. After cultivation at 37°C in the presence of 5% CO₂ for overnight, the cells were used for assay. For the sample buffer, HBSS supplemented with 0.1% BSA and 0.2 mM IBMX (Sigma) was used. The cells were washed twice with the sample buffer and pre-incubated at 37°C for 30 minutes. Thereafter, the cells were also washed twice and incubated at 37°C for 30 minutes after adding of sample. Farnesyl 2-phosphate or lysophophatidic acid was added to the sample to make a finalconcentration 2 µM. After incubation with the sample, a level of cAMP production was measured using cAMP Screen System (ABI). From the result, as shown in FIGs. 7 and 8, it was found that a level of cAMP production was increased in the manner specific to CHO-TGR4 when FPP or LPA was added.

### EXAMPLE 4

### Acquisition of rat-derived TGR4 receptor gene from rat brain cDNA by PCR method

Using rat brain cDNA as a template, and the following two primers, amplification by PCR was performed:

rF: 5'-GTCGACATGCCTCAGACTAGTTTCTCTCCCCAC-3' (SEQ ID NO: 12)

rR: 5'-GCTAGCCTTTCAGAGGGCTGAATCTTGGGGGCC-3' (SEQ ID NO:
13)

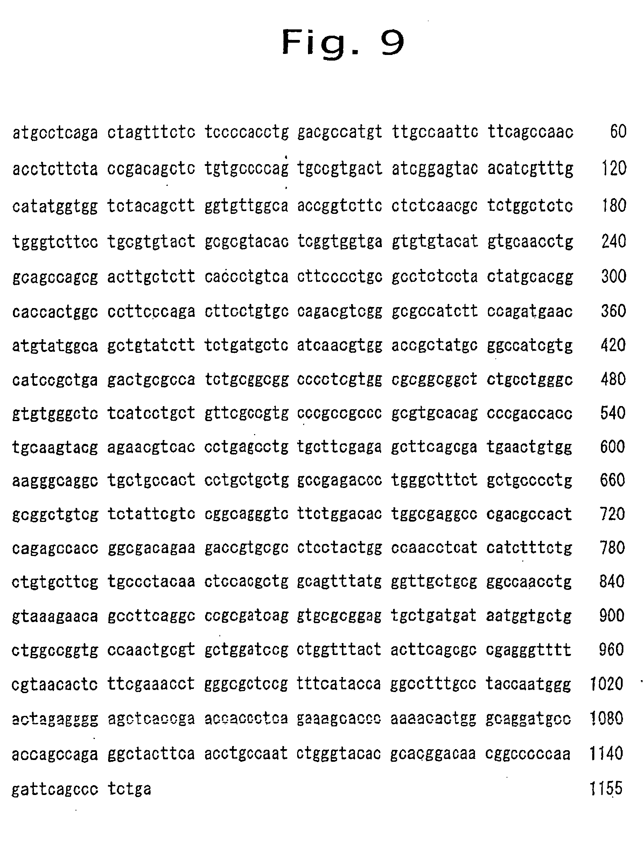

The reaction solution comprised of 1 µl of cDNA solution, 0.5 µl of rF (10 µM), 0.5 µl of rR (10 µM), 2.5µl of 10 x reaction solution attached, 2.5µl of dNTPs (10 mM), 0.5 µl of KlenTaq (CLONTECH) and 17.5 µl of distilled water supplied by Otsuka to make the total volume 25 µl. The PCR reaction was carried out using Thermal Cycler 9600 by heating of 95°C for 2 minutes, then a cycle set to include 98°C for 10 seconds followed by 60°C for 20 seconds and 72°C for 60 seconds, which was repeated 40 times. Using a portion of the PCR product, an amplification of the PCR product consisting of about 1000 bp was confirmed by electrophoresis. Then, the PCR product was purified using Qiagen PCR Purification Kit. Directly the sequencing was done, and a sequence shown in FIG 9, was obtained. An amino acid sequence deduced from the DNA sequence shown in FIG 9 was shown in FIG 10. Subsequently, the PCR product recovered from the gel was subcloned into Escherichia coli JM109 using the TA Cloning Kit (Invitrogen) to get Escherichia coli JM109/pTArTGR4. From Escherichia coli obtained by subcloning, plasmid pTArTGR4 was extracted with plasmid extraction instrument (Kurabo). A base sequence of inserted fragment was determined and was confirmed to be a rat-derived TGR4 gene. In addition, a homology between rat type and human type was given 79%.

### EXAMPLE 5

### Acquisition of mouse-derived TGR4 receptor gene from mouse brain cDNA by PCR method

Using mouse brain cDNA as a template, and the following two primers, amplification by PCR was performed:

mF: 5'-GTCGACATGCCTCAGACTAATTTCTCTTCCCAC-3' (SEQ ID NO:
14)

mR: 5'-GCTAGCTCAGAGAGCAGAATCCTGGGAGCAGTT-3' (SEQ ID NO:
15)

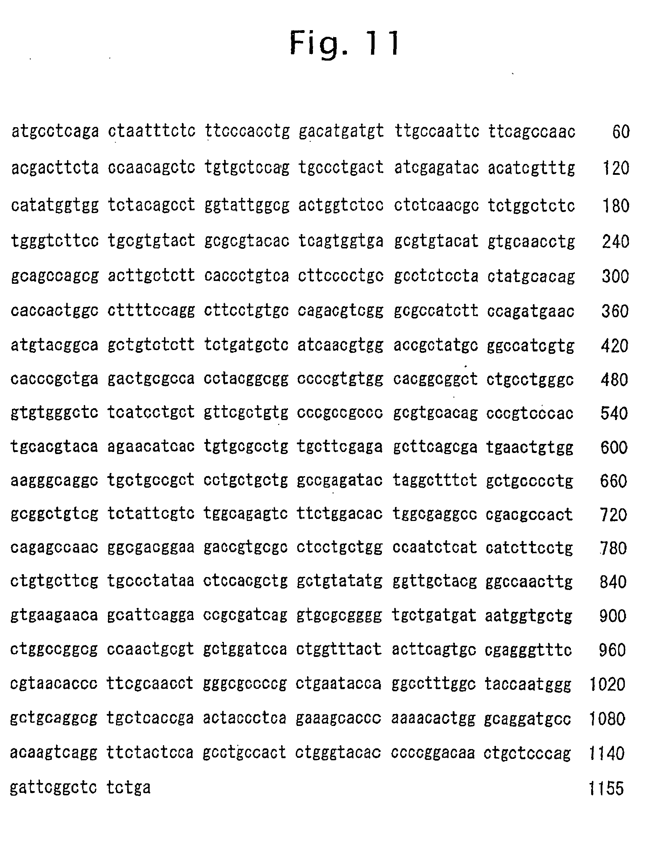

The reaction solution comprised of 1 µl of cDNA solution, 0.5 µl of mF (10 µM), 0.5 µl of mR (10 µM), 2.5µl of 10 x reaction solution attached, 2.5µl of dNTPs (10 mM), 0.5 µl of KlenTaq (CLONTECH) and 17.5 µl of distilled water supplied by Otsuka to make the total volume 25 µl. The PCR reaction was carried out using Thermal Cycler 9600 by heating of 95°C for 2 minutes, then a cycle set to include 98°C for 10 seconds followed by 60°C for 20 seconds and 72°C for 60 seconds, which was repeated 40 times. Using a portion of the PCR product, an amplification of the PCR product consisting of about 1000 bp was confirmed by electrophoresis. Then, the PCR product was purified using Qiagen PCR Purification Kit. Directly the sequencing was done, and a sequence shown in FIG 11 was obtained. An amino acid sequence deduced from the DNA sequence shown in FIG 11 was shown in FIG 12. Subsequently, the PCR product recovered from the gel was subcloned into Escherichia coli JM109 using the TA Cloning Kit (Invitrogen) to get Escherichia coli JM109/pTAmTGR4. From Escherichia coli obtained by subcloning, plasmid pTAmTGR4 was extracted with plasmid extraction instrument (Kurabo). A base sequence of inserted fragment was determined and was confirmed to be a rat-derived TGR4 gene. In addition, a homology between mouse type and human type was given 81%.

### EXAMPLE 6

### Intracellular translocation of TGR4-GFP fusion protein, which is expressed in CHO cells, by addition of farnesyl 2-phosphate

Expression plasmid wherein fusion protein that cDNA encoding Green Fluorescent protein (GFP) isolated from Aequorea coerulescens was ligated to the C-terminus of TGR4 by adjusting a translation frame, was constructed. As GFP cDNA, a fragment excised from GFP-expressing vector pQBI25 (Takara Shuzo) was used. The stop codon of TGR4 was modified to the recognition sequence of restriction enzyme NheI by the PCR method. GFP fragment was ligated to the terminus, and the obtained fragment was inserted into the expression vector pAKKO-111H. The thus obtained plasmid, which is an expression vector for fusion protein of TGR4 and GFP (hereinafter referred to as TGR4-GFP) was transfected to CHO-mock cells by the following method. The CHO-mock cells were suspended in growth medium (DMEM (Dulbecco's Modified Eagle Medium) (GIBCO BRL) supplemented with 10% fetal bovine serum (GIBCO BRL)), seeded on Lab-Tek II coverglass chamber (Nalgen Nunc), which has 4 chambers, at the concentration of 0.6 x 10⁵ cells/chamber. After cultivation at 37°C in the presence of 5% CO₂ for overnight, transfection was carried out. For transfection, Lipofectamine™ 2000 Reagent (GIBCO BRL) was used. Firstly, 2 µl of Lipofectamine™ 2000 Reagent and 50 µl of OPTI-MEM-I (GIBCO BRL) were admixed and stood for 20 minutes at room temperature to form a complex of DNA with lipofectamine. Then 100 µl of the above CHO cells were added to the cultured chamber. The culture was further performed at 37°C in the presence of 5% CO₂ for overnight. The medium was replaced with the medium for confocal microscopic observation (Hanks' Balanced Salt Solution (GIBCO BRL) supplemented with 0.1% bovine albumin (essentially Fatty Acid Free, GIBCO BRL). Then with confocal microscope (Leica), fluorescent image of GFP was observed. In this case, the excitation of GFP was performed at 488 nm.

As a result, TGR4-GFP fusion protein was observed in cell membrane. Thirty minutes after addition of farnesyl 2-phosphate to the medium to be 10⁻⁶ M, the GFP fluorescence was not found in cell membrane. It was found that the fluorescence was translocated to cytoplasm. This fact indicated that TGR4 is a G protein-coupled receptor, which is expressed in cell membrane and translocates to cytoplasm by reaction with farnesyl 2-phosphate, i.e., internalizes.

### EXAMPLE 7

### Analysis of expression distribution of human TGR4 mRNA

For quantification of mRNA expression level, ABI PRISM 7700 Sequence Detector (Applied Biosystems) was used. Primers and probe used for quantification of expression level were designed based on the base sequence of human TGR4 (SEQ ID NO: ) using software for ABI PRISM 7700 Sequence Detector, PrimerExpress (applied Biosystems). The cDNA for template was synthesized from 1 µg of polyA+ RNA derived from various human tissues using random primers at 42°C. For reverse trancription, Superscript II reversetranscriptase (GIBCO BRL) was used. The reaction was performed in accordance with the manual attached. After completion of the reaction, the product was precipitated with ethanol and dissolved in 100 µl. For fractionated cDNA from hemocyte, Multiple Tissue cDNA (MYTC™) panels Human Blood Fractions (CLONTECH) was used. The reaction solution for ABI PRISM 7700 Sequence Detector consisted of, according to the manual of TaqMan Universal PCR Master Mix (Applied Biosystems), 12.5 µl of Master Mix, 0.9 µM primers, 0.25 µM probe and 1 µl of cDNA solution of each sample, and filled up to 25 µl with distilled water. The reaction for ABI PRISM 7700 Sequence Detector was done under the following conditions: 50°C for 2 minutes, 95°C for 10 minutes, then a cycle set to include 95°C for 15 seconds and 60°C for 1 minute, which is repeated 40 times.

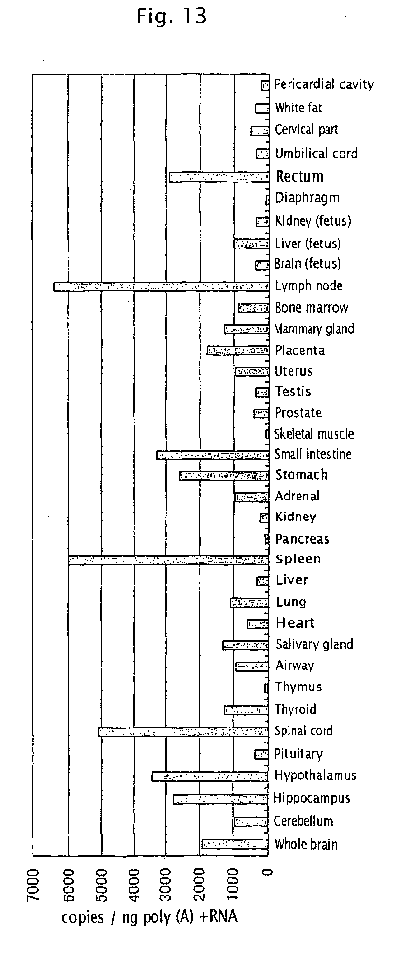

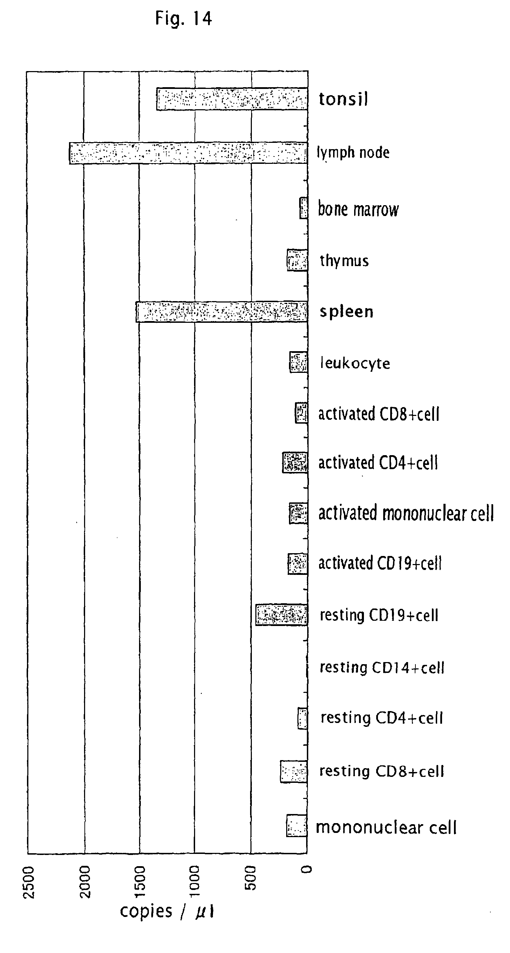

Expression distribution of TGR4 mRNA in various human tissues was shown in FIG. 13. In tissues associated with immunity such as spleen and lymph node, high expression was observed. In addition, an expression level of TGR4 mRNA in human hemocyte was shown in FIG. 14.

### EXAMPLE 8

### Analysis of expression distribution of rat TGR4 mRNA

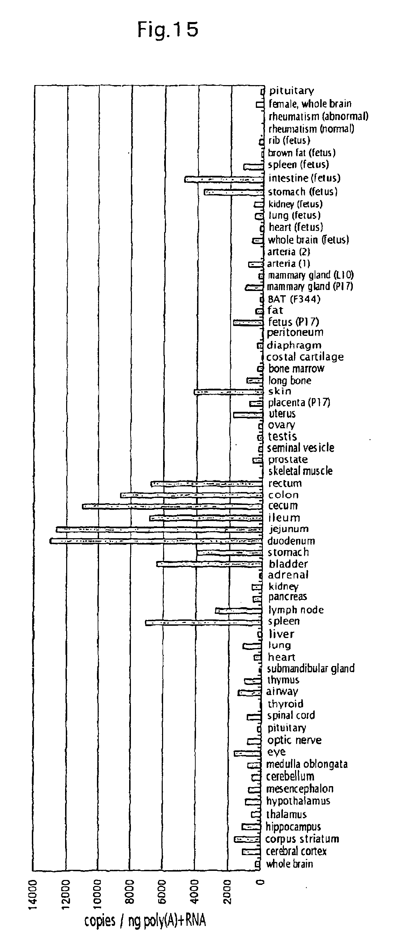

Various tissues were excised from Wistar rat and total RNA was prepared therefrom using Isogen (Nippon Gene). From total RNA obtained, poly(A)+ RNA was prepared with mRNA purification kit (Pharmacia). In each case, the preparation was carried out in accordance with each manual. One µg of poly(A)+ RNA obtained was treated with Dnase I (Amplification Grade, GIBCO BRL). Then, from the treated RNA equivalent to 160 ng, using RNA PCR kit (Takara), cDNA was synthesized at 42°C in accordance with manual. The synthesized cDNA was diluted to 4 ng/µl converted to poly(A)+ RNA, and used as a template for RT-PCR as stated below. RT-PCR was carried out with Sequence Detection System Prism 7700 (PE Biosystems). As primers for amplification and detection, 5'-CACCTGCAAGTACGAGAACGT-3'. (SEQ ID NO: 16) and 5'-TGCCCTTCCACAGTTCATC-3' (SEQ ID NO: 17) were used. And as TaqMan probe, 5'-(Fam)-TGAGCCTGTGCTTCGAGAGCTTCA-(Tamra)-3' (SEQ ID NO: 18) was used. The reaction solution for RT-PCR consisted of 12.5 µl of TaqMan Universal PCR Master Mix (PE Biosystems), 0.05 µl each of 100 µM primers, 0.5 µl of 5 µM TaqMan probe and 0.5 µl of cDNA solution prepared above to make total volume 25 µl by distilled water. The reaction was done under the following conditions: 50°C for 2 minutes, 95°C for 10 minutes, then a cycle set to include 95°C for 15 seconds and 60°C for 1 minute, which is repeated 40 times. An expression level of GPR7 ligand mRNA in rat-derived tissues was calculated as copy numbers per 1 ng of poly(A)+ RNA. As shown in FIG 15, rat-derived TGR4 mRNA was highly expressed in spleen, lymph node, and gut associated tissues, which are associated with immunity.

### EXAMPLE 9

### Activation of MAP kinase specific to TGR4 expressing CHO cells

Human TGR4-expressing CHO cells and mock cells were seeded on 6-well plate at the concentration of 3 x 10⁵ cells/well and cultured for overnight. For removal of serum, the medium was replaced with α-MEM including no nucleic acid supplemented with 0.1% BSA, and the cells were further incubated for overnight. Until just before assay, preincubation using the same medium for 2 hours was carried out. By adding 0.1 µM farnesyl 2-phosphate to the medium, the treatment was started. With respect to each given time, the medium was removed by aspiration and the reaction was terminated by addition of sumple buffer for SDS-PAGE. In accordance with conventional manner, SDS-PAGE was done and then the proteins were transferred to nitrocellulose membrane. Following the method described in the manual of Phospho Plus p44/42 MAPK (Thr202/Tyr204) Antibody Kit (Cell Signaling), detection of p44/42 MAP kinase was performed. As a result, phosphorylation of MAP kinase specific to human TGR-expressing CHO cells by treatment of farnesyl 2-phosphate was detected. Thus it became clear that the enzyme was activated.

### EXAMPLE 10

### Concentration dependency of intracellular calcium ion mobilization activity specific to TGR4 by substances associated with various phospholipids compounds

As well as the method described in EXAMPLE 7, using TGR4-expressing CHO cells and mock CHO cells, a study on concentration dependency of intracellular calcium ion mobilization activity by substances associated with various phospholipids compounds was made. For substances associated with various phospholipids compounds, geranylgeranyl 2-phosphate, farnesyl 2-phosphate, geranyl 2-phosphate, lysophosphatidic acid, sphingosine 1-phosphate, nordeoxycholic acid, deoxycholic acid, geranylgeranyol, farnesol were used. As shown in FIG 16, intracellular calcium ion mobilization activity dependent on concentration was perceived by adding phospholipids such as geranylgeranyl 2-phosphate, farnesyl 2-phosphate, or lysophosphatidic acid to CHO-TGR4. On the other hand, as shown in FIG 17, in the mock CHO cells, in which TGR4 is not expressed, no activity was detected. From these results, it became clear that the above-mentioned phospholipids compounds acts as an agonist specific to TGR4. In addition, EC₅₀ of cAMP production increasing activity for TGR4, which the related compounds including compounds other than those shown in the figure exhibit, was as shown in Table 1.

**[Table 1]**

| Compounds | Intracellular Ca²⁺ mobilization activity* | cAMP production increasing activity** |
|---|---|---|
| Dimethylaryl 2-phosphate | >2.5 | >50 |
| Geranylgeranyl2-phosphate | 0.019 | 3.5 |
| Farnesyl 2-phosphate | 0.029 | 3.1 |
| Geranyl 2-phosphate | >2.5 | >50 |
| Lysophosphatidic acid | 0.035 | >50 |
| Sphingosine 1-phosphate | 1.5 | >50 |
| Geranylgeranyol | >2.5 | >50 |
| Farnesol | >2.5 | >50 |
| Geranyol | >2.5 | >50 |
| Geranylamine | >2.5 | >50 |
| Geranyl acetic acid | >2.5 | >50 |
| Geranyl acetone | >2.5 | >50 |
| Geranylchloride | >2.5 | >50 |
| Nordeoxycholic acid | >2.5 | >50 |
| Deoxycholic acid | >2.5 | >50 |
| Lithocolic acid | >2.5 | >50 |

| | | |
|---|---|---|
| *: It is calculated, if assumed that the reaction when 2 x 10⁻⁶ M farnesyl 2-phosphate was added is equal to 100%. | | |
| **: It is calculated, if assumed that the reaction when 115 x 10⁻⁶ M farnesyl 2-phosphate was added is equal to 100%. | | |

### EXAMPLE 11

### Concentration dependency of cAMP production increasing activity specific to TGR4 by substances associated with various phospholipids compounds

As well as the method described in EXAMPLE 8, using TGR4-expressing CHO cells and mock CHO cells, a study on concentration dependency of cAMP production increasing activity by substances associated with various phospholipids compounds was made. As shown in FIG 18, intracellular calcium ion mobilization activity dependent on concentration was perceived by adding phospholipids such as geranylgeranyl 2-phosphate, farnesyl 2-phosphate, or lysophosphatidic acid to CHO-TGR4. For abbreviation of compounds, see EXAMPLE 9. On the other hand, as shown in FIG 19, in the mock CHO cells, in which TGR4 is not expressed, no activity was detected. From these results, it became clear that the above-mentioned phospholipids compounds acts as an agonist specific to TGR4. In addition, EC₅₀ of cAMP production increasing activity for TGR4, which the related compounds including compounds other than those shown in the figure exhibit, was as shown in Table 1 of EXAMPLE 10.

### INDUSTRIAL APPLICABILITY

By using the G protein-coupled receptor protein of the present invention, its partial peptides, or salts thereof and the phospholipids compound, which is a ligand to the protein, a compound that alters the binding property between phospholipids compound and the G protein-coupled receptor protein of the present invention, its partial peptides, or salts thereof can be screened efficiently.

## Claims

1. A screening method for (1) a G protein-coupled receptor protein containing the same or substantially the same amino acid sequence as the amino acid sequence represented by SEQ ID NO: 1, a partial peptide thereof or salts thereof, and (2) a compound or a salt thereof that alters the binding property between the receptor protein or a salt thereof and phopholipid compound.

2. The screening method according to claim 1, wherein the G protein-coupled receptor protein is a protein consisting of the amino acid sequence represented by SEQ ID NO: 1, SEQ ID NO: 8, or SEQ ID NO: 10.

3. The screening method according to claim 1, wherein the phospholipid compound is (1) a compound, wherein a pyrophosphate group is bound to the end of repeated structure consisting of at least one isoprene unit, or (2) a compound, wherein a phosphate group is bound to glycerol bone, and thereto a fatty acid or long chain alcohol is bound by ester bond.

4. The screening method according to claim 1, wherein the phospholipids compound is geranylgeranyl 2-phosphate (GGPP), farnesyl 2-phosphate (FPP) or lysophophatydinic acid (LPA).

5. The screening method according to claim 1, wherein the phospholipids compound is geranylgeranyl 2-phosphate (GGPP) or farnesyl 2-phosphate (FPP).

6. A screening kit for (1) a G protein-coupled receptor protein containing the same or substantially the same amino acid sequence as the amino acid sequence represented by SEQ ID NO: 1, a partial peptide thereof or salts thereof, and (2) a compound or a salt thereof that alters the binding property between the receptor protein or a salt thereof and phopholipid compound.

7. A compound or a salt thereof that alters the binding property between phopholipid compound and the receptor protein or a salt thereof containing the same or substantially the same amino acid sequence as the amino acid sequence represented by SEQ ID NO: 1, which is obtainable by using the screening method according to claim 1 or the screening kit according to claim 6.

8. A medicine comprising the compound or a salt thereof that alters the binding property between phopholipid compound and the receptor protein or a salt thereof containing the same or substantially the same amino acid sequence as the amino acid sequence represented by SEQ ID NO: 1.

9. The medicine according to claim 8, which is a prophylactic/therapeutic agent for central nerve diseases, inflammatory diseases, circulatory diseases, cancer, diabetes, immune system diseases or alimentary diseases.

10. The medicine according to claim 8, which is a prophylactic/therapeutic agent for liver and cholecyst diseases, alimentary diseases, anxiety, pain or obesity.

11. A determination method of ligand to the G protein-coupled receptor protein or its salt, which comprises measuring intracellular Ca²⁺ concentration increasing activity or intracellular cAMP producing activity when a test compound is brought into contact with a cell containing the G protein-coupled receptor protein containing the same or substantially the same amino acid sequence as the amino acid sequence represented by SEQ ID NO: 1.

12. A ligand, which is obtained by the method according to claim 11.

13. A screening method of agonist or antagonist to the G protein-coupled receptor protein or its salt containing the same or substantially the same amino acid sequence as the amino acid sequence represented by SEQ ID NO: 1, which comprises comparing (i) the case where the G protein-coupled receptor protein containing the same or substantially the same amino acid sequence as the amino acid sequence represented by SEQ ID NO: 1, a partial peptide thereof or salts thereof are brought into contact with (a) phospholipids compound or (b) compound or its salt that alters the binding property between the phospholipids compound and the G protein-coupled receptor protein or its salt, and (ii) the case where the G protein-coupled receptor protein containing the same or substantially the same amino acid sequence as the amino acid sequence represented by SEQ ID NO: 1, a partial peptide thereof or salts thereof are brought into contact with (a) phospholipids compound or (b) compound or its salt that alters the binding property between the phospholipids compound and the G protein-coupled receptor protein or its salt, and a test compound.

14. A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to the G protein-coupled receptor protein, a partial peptide thereof or salts thereof in the case where (i) (a) labeled phospholipids compound or (b) labeled compound or its salt that alters the binding property between the phospholipids compound and the G protein-coupled receptor protein or its salt is brought into contact with the G protein-coupled receptor protein, a partial peptide thereof or salts thereof, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein, a partial peptide thereof or salts thereof.

15. A screening method of agonist or antagonist to the G protein-coupled receptor protein or its salt containing the same or substantially the same ammo acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to a cell containing the G protein-coupled receptor protein, in the case where (i) (a) labeled phospholipids compound or (b) labeled compound or its salt that alters the binding property between the phospholipids compound and the G protein-coupled receptor protein or its salt is brought into contact with the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the cell containing the G protein-coupled receptor protein.

16. A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to a membrane fraction of the cell containing the G protein-coupled receptor protein, in the case where (i) (a) labeled phospholipids compound or (b) labeled compound or its salt that alters the binding property between the phospholipids compound and the G protein-coupled receptor protein or its salt is brought into contact with the membrane fraction of the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the membrane fraction of the cell containing the G protein-coupled receptor protein.

17. A screening method of agonist or antagonist to the G protein-coupled receptor protein or its salt containing the same or substantially the same amino acid sequence as the amino acid sequence represented by SEQ ID NO: 1, which comprises measuring and comparing the binding amount of (a) labeled phospholipids compound or (b) labeled compound or its salt that alters the binding property between the phospholipids compound and the G protein-coupled receptor protein or its salt to the G protein-coupled receptor protein, in the case where (i) (a) labeled phospholipids compound or (b) labeled compound or its salt that alters the binding property between the phospholipids compound and the G protein-coupled receptor protein or its salt is brought into contact with the G protein-coupled receptor protein expressing on cell membrane of a transformant, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein expressing on cell membrane of the transformant.

18. A screening method of agonist or antagonist to the G protein-coupled receptor protein or its salt containing the same or substantially the same amino acid sequence as the amino acid sequence represented by SEQ ID NO: 1, which comprises measuring and comparing intracellular Ca²⁺ concentration increasing activity or intracellular cAMP producing activity, in the case where (i) (a) labeled phospholipids compound or (b) labeled compound or its salt that alters the binding property between the phospholipids compound and the G protein-coupled receptor protein or its salt is brought into contact with the cell containing the G protein-coupled receptor protein, and (ii) (a) labeled phospholipids compound or (b) labeled compound or its salt that alters the binding property between the phospholipids compound and the G protein-coupled receptor protein or its salt, and a test compound are brought into contact with the cell containing the G protein-coupled receptor protein.

19. A screening method of agonist or antagonist to the G protein-coupled receptor protein or its salt containing the same or substantially the same amino acid sequence as the amino acid sequence represented by SEQ ID NO: 1, which comprises measuring and comparing intracellular Ca²⁺ concentration increasing activity or intracellular cAMP producing activity, in the case where (i) (a) labeled phospholipids compound or (b) labeled compound or its salt that alters the binding property between the phospholipids compound and the G protein-coupled receptor protein or its salt is brought into contact with the G protein-coupled receptor protein expressing on cell membrane of a transformant, and (ii) (a) labeled phospholipids compound or (b) labeled compound or its salt that alters the binding property between the phospholipids compound and the G protein-coupled receptor protein or its salt, and a test compound are brought into contact with the G protein-coupled receptor protein expressing on cell membrane of the transformant.

20. The screening method according to claim 1, wherein the phospholipid compound is (1) a compound, wherein a pyrophosphate group is bound to the end of repeated structure consisting of at least one isoprene unit, or (2) a compound, wherein a phosphate group is bound to glycerol bone, and thereto a fatty acid or long chain alcohol is bound by ester bond.

21. The screening method according to claims 13 through 20, wherein the phospholipids compound is geranylgeranyl 2-phosphate (GGPP), farnesyl 2-phosphate (FPP) or lysophophatidic acid (LPA).

22. The screening method according to claims 13 through 20, wherein the phospholipids compound is geranylgeranyl 2-phosphate (GGPP) or farnesyl 2-phosphate (FPP).

23. The screening method according to claims 13 through 20, wherein the test compound is a compound designed for binding to the ligand binding pocket based on atom coordinate of the active site of the G protein-coupled receptor protein or its salt containing the same or substantially the same amino acid sequence as the amino acid sequence represented by SEQ ID NO: 1 and the location of ligand binding pocket.

24. An agonist or an antagonist to the G protein-coupled receptor protein containing the same or substantially the same amino acid sequence as the sequence represented by SEQ ID NO: 1, or a salt thereof, which is obtainabie using any screening method according to claims 13 through 23.

25. A medicine, which comprises the agonist or the antagonist to the G protein-coupled receptor protein containing the same or substantially the same amino acid sequence as the sequence represented by SEQ ID NO: 1.

26. A prophylactic/therapeutic agent for central nerve diseases, inflammatory diseases, circulatory diseases, cancer, diabetes, immune system diseases or alimentary diseases, which comprises the agonist or the antagonist to the G protein-coupled receptor protein containing the same or substantially the same amino acid sequence as the sequence represented by SEQ ID NO: 1.

27. A prophylactic/therapeutic agent for liver and cholecyst diseases, alimentary diseases, anxiety, pain or obesity, which comprises the agonist or the antagonist to the G protein-coupled receptor protein containing the same or substantially the same amino acid sequence as the sequence represented by SEQ ID NO: 1.

28. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises the G protein-coupled receptor protein containing the same or substantially the same amino acid sequence as the sequence represented by SEQ ID NO: 1, a partial peptide thereof or salts thereof.

29. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a DNA containing DNA encoding the G protein-coupled receptor protein containing the same or substantially the same amino acid sequence as the sequence represented by SEQ ID NO: 1.

30. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a polynucleotide containing polynucleotide encoding the G protein-coupled receptor protein containing the same or substantially the same amino acid sequence as the sequence represented by SEQ ID NO: 1.

31. A diagnostic method for central nerve diseases, inflammatory diseases, circulatory diseases, cancer, diabetes, immune system diseases, liver and cholecyst diseases, alimentary diseases, anxiety, pain or obesity, which comprises using a polynucleotide containing polynucleotide encoding the G protein-coupled receptor protein containing the same or substantially the same amino acid sequence as the sequence represented by SEQ ID NO: 1, or a partial peptide thereof.

32. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a compound that alters an expression level of the G protein-coupled receptor protein containing the same or substantially the same amino acid sequence as the sequence represented by SEQ ID NO: 1, or a salt thereof.

33. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises an antibody against the G protein-coupled receptor protein containing the same or substantially the same amino acid sequence as the sequence represented by SEQ ID NO: 1, a partial peptide thereof or salts thereof.

34. A diagnostic agent for central nerve diseases, inflammatory diseases, circulatory diseases, cancer, diabetes, immune system diseases, liver and cholecyst diseases, alimentary diseases, anxiety, pain or obesity, which comprises an antibody against the G protein-coupled receptor protein containing the same or substantially the same amino acid sequence as the sequence represented by SEQ ID NO: 1, a partial peptide thereof or salts thereof.

35. A diagnostic method for central nerve diseases, inflammatory diseases, circulatory diseases, cancer, diabetes, immune system diseases, liver and cholecyst diseases, alimentary diseases, anxiety, pain or obesity, which comprises using the quantification method for the G protein-coupled receptor protein using the antibody against the G protein-coupled receptor protein containing the same or substantially the same amino acid sequence as the sequence represented by SEQ ID NO: 1, a partial peptide thereof or salts thereof.

36. A prophylactic/therapeutic agent for central nerve diseases, inflammatory diseases, circulatory diseases, cancer, diabetes, immune system diseases, liver and cholecyst diseases, alimentary diseases, anxiety, pain or obesity, which comprises a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof.

37. A prophylactic/therapeutic method for central nerve diseases, inflammatory diseases, circulatory diseases, cancer, diabetes, immune system diseases, liver and cholecyst diseases, alimentary diseases, anxiety, pain or obesity, which comprises administering to mammals an effective amount of (a) the agonist or the antagonist to the G protein-coupled receptor protein containing the same or substantially the same amino acid sequence as the sequence represented by SEQ ID NO: 1, or a salt thereof, (b) the G protein-coupled receptor protein containing the same or substantially the same amino acid sequence as the sequence represented by SEQ ID NO: 1, a partial peptide thereof or salts thereof, (c) a polynucleotide containing a polynucleotide encoding the G protein-coupled receptor protein containing the same or substantially the same amino acid sequence as the sequence represented by SEQ ID NO: 1, or a partial peptide thereof, (d) an antibody against the G protein-coupled receptor protein containing the same or substantially the same amino acid sequence as the sequence represented by SEQ ID NO: 1, a partial peptide thereof or salts thereof, or (e) a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof.

38. Use of (a) the agonist or the antagonist to the G protein-coupled receptor protein containing the same or substantially the same amino acid sequence as the sequence represented by SEQ ID NO: 1, or a salt thereof, (b) the G protein-coupled receptor protein containing the same or substantially the same amino acid sequence as the sequence represented by SEQ ID NO: 1, a partial peptide thereof or salts thereof, (c) a polynucleotide containing a polynucleotide encoding the G protein-coupled receptor protein containing the same or substantially the same amino acid sequence as the sequence represented by SEQ ID NO: 1, or a partial peptide thereof, (d) an antibody against the G protein-coupled receptor protein containing the same or substantially the same amino acid sequence as the sequence represented by SEQ ID NO: 1, a partial peptide thereof or salts thereof, or (e) a polynucleotide comprising a complementary base sequence or a portion thereof to a polynucleotide containing a polypeptide encoding the G protein-coupled receptor protein containing the same or substantially the same amino acid sequence as the sequence represented by SEQ ID NO: 1, or a partial peptide thereof for manufacturing a prophylactic/therapeutic agent for central nerve diseases, inflammatory diseases, circulatory diseases, cancer, diabetes, immune system diseases, liver and cholecyst diseases, alimentary diseases, anxiety, pain or obesity.

39. A G protein-coupled receptor protein consisting of the amino acid sequence represented by SEQ ID NO: 8 or SEQ ID NO: 10, a partial peptide thereof or salts thereof.

40. A polynucleotide containing the polypeptide encoding the G protein-coupled receptor protein according to claim 39.

41. A DNA consisting of the base sequence represented by SEQ 8 or SEQ ID NO: 10.

42. A recombinant vector containing the polynucleotide according to claim 40.

43. A transformant, which is transformed with the recombinant vector according to claim 42.

44. A manufacturing method of the G protein-coupled receptor protein according to claim 39, or a salt thereof, which comprises culturing the transformant according to claim 43 and producing the G protein-coupled receptor protein according to claim 39, or a salt thereof.

45. A medicine, which comprises the G protein-coupled receptor protein according to claim 39, a partial peptide thereof or salts thereof.

46. A medicine, which comprises the polynucleotide according to claim 40.

47. A diagnostic agent, which comprises the polynucleotide according to claim 40.

48. An antibody against the G protein-coupled receptor protein according to claim 39, a partial peptide thereof or salts thereof.

49. The antibody according to claim 48, which is a neutralizing antibody that inactivates signal transduction of the G protein-coupled receptor protein according to claim 39.

50. A diagnostic agent, which comprises the antibody according to claim 48.

51. A medicine, which comprises the antibody according to claim 48.

52. An antisense polynucleotide, which comprises a complementary base sequence to the polynucleotide according to claim 40 or a portion thereof.

53. A diagnostic agent, which comprises the antisense polynucleotide according to claim 52.

54. A medicine, which comprises the antisense polynucleotide according to claim 52.

55. A screening method for the agonist or the antagonist to the G protein-coupled receptor protein according to claim 39, which comprises using the G protein-coupled receptor protein according to claim 39, a partial peptide thereof or salts thereof.

56. A screening kit for the agonist or the antagonist to the G protein-coupled receptor protein according to claim 39, which comprises the G protein-coupled receptor protein according to claim 39, a partial peptide thereof or salts thereof.

57. An agonist or an antagonist to the G protein-coupled receptor protein according to claim 39, which is obtained using the screening method according to claim 55 or the screening kit according to claim 56.

58. A medicine, which comprises an agonist or an antagonist to the G protein-coupled receptor protein according to claim 39.

59. A screening method for a compound that alters an expression level of the G protein-coupled receptor protein according to claim 39, or a salt thereof, which comprises using the polynucleotide according to claim 40.

60. A screening kit for a compound that alters an expression level of the G protein-coupled receptor protein according to claim 39, or a salt thereof, which comprises the polynucleotide according to claim 40.

61. A compound that alters an expression level of the G protein-coupled receptor protein according to claim 39, or a salt thereof, which is obtained using the screening method according to claim 59 or the screening kit according to claim 60.

62. A medicine, which comprises a compound that alters an expression level of the G protein-coupled receptor protein according to claim 39, or a salt thereof.


SEQUENCE LISTING

<110> Takeda Chemical Industries, Ltd.

<120> Screening Method

<130> 2946WOOP

<150> JP 2001-258479
<151> 2001-08-28

<160> 18

<210> 1
<211> 372
<212> PRT
<213> Homo Sapiens

<400> 1

<210> 2
<211> 1116
<212> DNA
<213> Homo Sapiens

<400> 2
atgttagcca acagctcctc aaccaacagt tctgttctcc cgtgtcctga ctaccgacct 60
acccaccgcc tgcacttggt ggtctacagc ttggtgctgg ctgccgggct ccccctcaac 120
gcgctagccc tctgggtctt cctgcgcgcg ctgcgcgtgc actcggtggt gagcgtgtac 180
atgtgtaacc tggcggccag cgacctgctc ttcaccctct cgctgcccgt tcgtctctcc 240
tactacgcac tgcaccactg gcccttcccc gacctcctgt gccagacgac gggcgccatc 300
ttccagatga acatgtacgg cagctgcatc ttcctgatgc tcatcaacgt ggaccgctac 360
gccgccatcg tgcacccgct gcgactgcgc cacctgcggc ggccccgcgt ggcgcggctg 420
ctctgcctgg gcgtgtgggc gctcatcctg gtgtttgccg tgcccgccgc ccgcgtgcac 480
aggccctcgc gttgccgcta ccgggacctc gaggtgcgcc tatgcttcga gagcttcagc 540
gacgagctgt ggaaaggcag gctgctgccc ctcgtgctgc tggccgaggc gctgggcttc 600
ctgctgcccc tggcggcggt ggtctactcg tcgggccgag tcttctggac gctggcgcgc 660
cccgacgcca cgcagagcca gcggcggcgg aagaccgtgc gcctcctgct ggctaacctc 720
gtcatcttcc tgctgtgctt cgtgccctac aacagcacgc tggcggtcta cgggctgctg 780
cggagcaagc tggtggcggc cagcgtgcct gcccgcgatc gcgtgcgcgg ggtgctgatg 840
gtgatggtgc tgctggccgg cgccaactgc gtgctggacc cgctggtgta ctactttagc 900
gccgagggct tccgcaacac cctgcgcggc ctgggcactc cgcaccgggc caggacctcg 960
gccaccaacg ggacgcgggc ggcgctcgcg caatccgaaa ggtccgccgt caccaccgac 1020
gccaccaggc cggatgccgc cagtcagggg ctgctccgac cctccgactc ccactctctg 1080
tcttccttca cacagtgtcc ccaggattcc gccctc 1116

<210> 3
<211> 35
<212> DNA
<213> Artificial Sequence

<220>
<223> Primer

<400> 3
gggtcgacat gttagccaac agctcctcaa ccaac 35

<210> 4
<211> 33
<212> DNA
<213> Artificial Sequence

<220>
<223> Primer

<400> 4
ggactagttc agagggcgga atcctgggga cac 33

<210> 5
<211> 28
<212> DNA
<213> Artificial Sequence

<220>
<223> Probe

<400> 5
ttcgtctctc ctactacgca ctgcacca 28

<210> 6
<211> 22
<212> DNA
<213> Artificial Sequence

<220>
<223> Primer

<400> 6

ggtggtgagc gtgtacatgt gt 22

<210> 7
<211> 22
<212> DNA
<213> Artificial Sequence

<220>
<223> Primer

<400> 7
catgttcatc tggaagatgg cg 22

<210> 8
<211> 384
<212> PRT
<213> Rattus spp

<400> 8
Met Pro Gln Thr Ser Phe Ser Pro His Leu Asp Ala Met Phe Ala Asn
                5                   10                  15
Ser Ser Ala Asn Thr Ser Ser Thr Asp Ser Ser Val Pro Gln Cys Arg
            20                  25                  30
Asp Tyr Arg Ser Thr His Arg Leu His Met Val Val Tyr Ser Leu Val
        35                  40                  45
Leu Ala Thr Gly Leu Pro Leu Asn Ala Leu Ala Leu Trp Val Phe Leu
    50                  55                  60
Arg Val Leu Arg Val His Ser Val Val Ser Val Tyr Met Cys Asn Leu
65                  70                  75                  80
Ala Ala Ser Asp Leu Leu Phe Thr Leu Ser Leu Pro Leu Arg Leu Ser
                85                  90                  95
Tyr Tyr Ala Arg His His Trp Pro Phe Pro Asp Phe Leu Cys Gln Thr
            100                 105                 110
Ser Gly Ala Ile Phe Gln Met Asn Met Tyr Gly Ser Cys Ile Phe Leu
        115                 120                 125
Met Leu Ile Asn Val Asp Arg Tyr Ala Ala Ile Val His Pro Leu Arg
    130                 135                 140
Leu Arg His Leu Arg Arg Pro Leu Val Ala Arg Arg Leu Cys Leu Gly
145                 150                 155                 160
Val Trp Ala Leu Ile Leu Leu Phe Ala Val Pro Ala Ala Arg Val His
                165                 170                 175
Ser Pro Thr Thr Cys Lys Tyr Glu Asn Val Thr Leu Ser Leu Cys Phe
            180                 185                 190
Glu Ser Phe Ser Asp Glu Leu Trp Lys Gly Arg Leu Leu Pro Leu Leu
        195                 200                 205
Leu Leu Ala Glu Thr Leu Gly Phe Leu Leu Pro Leu Ala Ala Val Val
    210                 215                 220
Tyr Ser Ser Gly Arg Val Phe Trp Thr Leu Ala Arg Pro Asp Ala Thr
225                 230                 235                 240
Gln Ser His Arg Arg Gln Lys Thr Val Arg Leu Leu Leu Ala Asn Leu
                245                 250                 255
Ile Ile Phe Leu Leu Cys Phe Val Pro Tyr Asn Ser Thr Leu Ala Val
            260                 265                 270
Tyr Gly Leu Leu Arg Ala Asn Leu Val Lys Asn Ser Leu Gln Ala Arg
        275                 280                 285
Asp Gln Val Arg Gly Val Leu Met Ile Met Val Leu Leu Ala Gly Ala
    290                 295                 300
Asn Cys Val Leu Asp Pro Leu Val Tyr Tyr Phe Ser Ala Glu Gly Phe
305                 310                 315                 320
Arg Asn Thr Leu Arg Asn Leu Gly Ala Pro Phe His Thr Arg Pro Leu
                325                 330                 335
Pro Thr Asn Gly Thr Arg Gly Glu Leu Thr Glu Pro Pro Ser Glu Ser
            340                 345                 350
Thr Gln Asn Thr Gly Gln Asp Ala Thr Ser Gln Arg Leu Leu Gln Pro
        355                 360                 365
Ala Asn Leu Gly Thr Arg Thr Asp Asn Gly Pro Gln Asp Ser Ala Leu
    370                 375                 380

<210> 9
<211> 1152
<212> DNA
<213> Rattus spp

<400> 9
atgcctcaga ctagtttctc tccccacctg gacgccatgt ttgccaattc ttcagccaac 60
acctcttcta ccgacagctc tgtgccccag tgccgtgact atcggagtac acatcgtttg 120
catatggtgg tctacagctt ggtgttggca accggtcttc ctctcaacgc tctggctctc 180
tgggtcttcc tgcgtgtact gcgcgtacac tcggtggtga gtgtgtacat gtgcaacctg 240
gcagccagcg acttgctctt caccctgtca cttcccctgc gcctctccta ctatgcacgg 300
caccactggc ccttcccaga cttcctgtgc cagacgtcgg gcgccatctt ccagatgaac 360
atgtatggca gctgtatctt tctgatgctc atcaacgtgg accgctatgc ggccatcgtg 420
catccgctga gactgcgcca tctgcggcgg cccctcgtgg cgcggcggct ctgcctgggc 480
gtgtgggctc tcatcctgct gttcgccgtg cccgccgccc gcgtgcacag cccgaccacc 540
tgcaagtacg agaacgtcac cctgagcctg tgcttcgaga gcttcagcga tgaactgtgg 600
aagggcaggc tgctgccact cctgctgctg gccgagaccc tgggctttct gctgcccctg 660
gcggctgtcg tctattcgtc cggcagggtc ttctggacac tggcgaggcc cgacgccact 720
cagagccacc ggcgacagaa gaccgtgcgc ctcctactgg ccaacctcat catctttctg 780
ctgtgcttcg tgccctacaa ctccacgctg gcagtttatg ggttgctgcg ggccaacctg 840
gtaaagaaca gccttcaggc ccgcgatcag gtgcgcggag tgctgatgat aatggtgctg 900
ctggccggtg ccaactgcgt gctggatccg ctggtttact acttcagcgc cgagggtttt 960
cgtaacactc ttcgaaacct gggcgctccg tttcatacca ggcctttgcc taccaatggg 1020
actagagggg agctcaccga accaccctca gaaagcaccc aaaacactgg gcaggatgcc 1080
accagccaga ggctacttca acctgccaat ctgggtacac gcacggacaa cggcccccaa 1140
gattcagccc tc 1152

<210> 10
<211> 384
<212> PRT
<213> Mus spp

<400> 10
Met Pro Gln Thr Asn Phe Ser Ser His Leu Asp Met Met Phe Ala Asn
                5                   10                  15
Ser Ser Ala Asn Thr Thr Ser Thr Asn Ser Ser Val Leu Gln Cys Pro
            20                  25                  30
Asp Tyr Arg Asp Thr His Arg Leu His Met Val Val Tyr Ser Leu Val
        35                  40                  45
Leu Ala Thr Gly Leu Pro Leu Asn Ala Leu Ala Leu Trp Val Phe Leu
    50                  55                  60
Arg Val Leu Arg Val His Ser Val Val Ser Val Tyr Met Cys Asn Leu
65                  70                  75                  80
Ala Ala Ser Asp Leu Leu Phe Thr Leu Ser Leu Pro Leu Arg Leu Ser
                85                  90                  95
Tyr Tyr Ala Gln His His Trp Pro Phe Pro Gly Phe Leu Cys Gln Thr
            100                 105                 110
Ser Gly Ala Ile Phe Gln Met Asn Met Tyr Gly Ser Cys Leu Phe Leu
        115                 120                 125
Met Leu Ile Asn Val Asp Arg Tyr Ala Ala Ile Val His Pro Leu Arg
    130                 135                 140
Leu Arg His Leu Arg Arg Pro Arg Val Ala Arg Arg Leu Cys Leu Gly
145                 150                 155                 160
Val Trp Ala Leu Ile Leu Leu Phe Ala Val Pro Ala Ala Arg Val His
                165                 170                 175
Ser Pro Ser His Cys Thr Tyr Lys Asn Ile Thr Val Arg Leu Cys Phe
            180                 185                 190
Glu Ser Phe Ser Asp Glu Leu Trp Lys Gly Arg Leu Leu Pro Leu Leu
        195                 200                 205
Leu Leu Ala Glu Ile Leu Gly Phe Leu Leu Pro Leu Ala Ala Val Val
    210                 215                 220
Tyr Ser Ser Gly Arg Val Phe Trp Thr Leu Ala Arg Pro Asp Ala Thr
225                 230                 235                 240
Gln Ser Gln Arg Arg Arg Lys Thr Val Arg Leu Leu Leu Ala Asn Leu
                245                 250                 255
Ile Ile Phe Leu Leu Cys Phe Val Pro Tyr Asn Ser Thr Leu Ala Val
            260                 265                 270
Tyr Gly Leu Leu Arg Ala Asn Leu Val Lys Asn Ser Ile Gln Asp Arg

275                 280                 285
Asp Gln Val Arg Gly Val Leu Met Ile Met Val Leu Leu Ala Gly Ala
    290                 295                 300
Asn Cys Val Leu Asp Pro Leu Val Tyr Tyr Phe Ser Ala Glu Gly Phe
305                 310                 315                 320
Arg Asn Thr Leu Arg Asn Leu Gly Ala Pro Leu Asn Thr Arg Pro Leu
                325                 330                 335
Ala Thr Asn Gly Ala Ala Gly Val Leu Thr Glu Leu Pro Ser Glu Ser
            340                 345                 350
Thr Gln Asn Thr Gly Gln Asp Ala Thr Ser Gln Val Leu Leu Gln Pro
        355                 360                 365
Ala Thr Leu Gly Thr Pro Pro Asp Asn Cys Ser Gln Asp Ser Ala Leu
    370                 375                 380

<210> 11
<211> 1152
<212> DNA
<213> Mus spp

<400> 11
atgcctcaga ctaatttctc ttcccacctg gacatgatgt ttgccaattc ttcagccaac    60
acgacttcta ccaacagctc tgtgctccag tgccctgact atcgagatac acatcgtttg   120
catatggtgg tctacagcct ggtattggcg actggtctcc ctctcaacgc tctggctctc   180
tgggtcttcc tgcgtgtact gcgcgtacac tcagtggtga gcgtgtacat gtgcaacctg   240
gcagccagcg acttgctctt caccctgtca cttcccctgc gcctctccta ctatgcacag   300
caccactggc cttttccagg cttcctgtgc cagacgtcgg gcgccatctt ccagatgaac   360
atgtacggca gctgtctctt tctgatgctc atcaacgtgg accgctatgc ggccatcgtg   420
cacccgctga gactgcgcca cctacggcgg ccccgtgtgg cacggcggct ctgcctgggc   480
gtgtgggctc tcatcctgct gttcgctgtg cccgccgccc gcgtgcacag cccgtcccac   540
tgcacgtaca agaacatcac tgtgcgcctg tgcttcgaga gcttcagcga tgaactgtgg   600
aagggcaggc tgctgccgct cctgctgctg gccgagatac taggctttct gctgcccctg   660
gcggctgtcg tctattcgtc tggcagagtc ttctggacac tggcgaggcc cgacgccact   720
cagagccaac ggcgacggaa gaccgtgcgc ctcctgctgg ccaatctcat catcttcctg   780
ctgtgcttcg tgccctataa ctccacgctg gctgtatatg ggttgctacg ggccaacttg   840
gtgaagaaca gcattcagga ccgcgatcag gtgcgcgggg tgctgatgat aatggtgctg   900
ctggccggcg ccaactgcgt gctggatcca ctggtttact acttcagtgc cgagggtttc   960
cgtaacaccc ttcgcaacct gggcgccccg ctgaatacca ggcctttggc taccaatggg  1020
gctgcaggcg tgctcaccga actaccctca gaaagcaccc aaaacactgg gcaggatgcc  1080
acaagtcagg ttctactcca gcctgccact ctgggtacac ccccggacaa ctgctcccag  1140
gattcggctc tc                                                      1152

<210> 12
<211> 33
<212> DNA
<213> Artificial Sequence

<220>
<223> Primer

<400> 12
gtcgacatgc ctcagactag tttctctccc cac       33

<210> 13
<211> 33
<212> DNA
<213> Artificial Sequence

<220>
<223> Primer

<400> 13
gctagccttt cagagggctg aatcttgggg gcc       33

<210> 14
<211> 33
<212> DNA
<213> Artificial Sequence

<220>
<223> Primer

<400> 14
gtcgacatgc ctcagactaa tttctcttcc cac 33

<210> 15
<211> 33
<212> DNA
<213> Artificial Sequence

<220>
<223> Primer

<400> 15
gctagctcag agagcagaat cctgggagca gtt 33

<210> 16
<211> 21
<212> DNA
<213> Artificial Sequence

<220>
<223> Primer

<400> 16
cacctgcaag tacgagaacg t 21

<210> 17
<211> 19
<212> DNA
<213> Artificial Sequence

<220>
<223> Primer

<400> 17
tgcccttcca cagttcatc 19

<210> 18
<211> 24
<212> DNA
<213> Artificial Sequence

<220>
<223> Probe

<400> 18
tgagcctgtg cttcgagagc ttca 24